# EUROPÄISCHE PATENTANMELDUNG

(11) **EP 2 080 758 A2**
(43) Veröffentlichungstag der Anmeldung: **22.07.2009**
(21) Anmeldenummer: 07121882.0
(22) Anmeldetag: 29.11.2007
(51) Int. Cl.: C07D 401/10, A01N 43/40

(54) **Halogen substituierte delta-1-Pyrroline**

(71) Anmelder: Bayer CropScience AG, 40789 Monheim (DE)
(72) Erfinder: Die Erfindernennung liegt noch nicht vor

(57) **Zusammenfassung**

Halogensubstituierte Δ¹-Pyrroline der Formel (I) 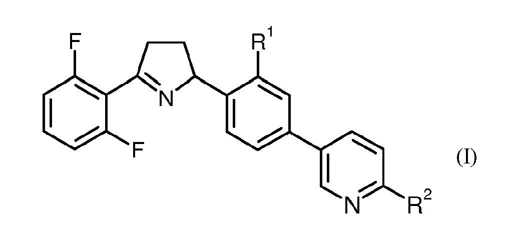 in welcher
R¹, R² die in der Beschreibung angegebenen Bedeutungen hat,
mehrere Verfahren zur Herstellung dieser Stoffe und deren Verwendung zur Bekämpfung von Schädlingen.

## Beschreibung

Die vorliegende Erfindung betrifft neue Δ¹-Pyrroline, mehrere Verfahren zu ihrer Herstellung und ihre Verwendung als Schädlingsbekämpfungsmittel.

Es ist bereits bekannt, dass zahlreiche Δ¹-Pyrroline insektizide Eigenschaften besitzen (vgl. WO 98/22438, WO 02/064588, WO 99/59968, WO 99/59967, WO 02/24646, WO 02/046151).
Die Wirksamkeit dieser Stoffe ist gut, lässt aber in manchen Fällen zu wünschen übrig.
Es wurden nun neue Δ¹-Pyrroline der Formel (I) 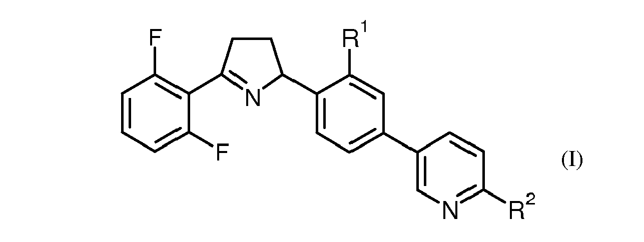 gefunden, in welcher
R¹ für Halogen steht
R² für jeweils gegebenenfalls substituiertes Alkyl, Alkenyl, Alkoxy, Alkenyloxy; oder für jeweils gegebenenfalls substituiertes Cycloalkyl, Cycloalkoxy steht.

Die Verbindungen der Formel (I) können gegebenenfalls in Abhängigkeit von der Art und Anzahl der Substituenten als geometrische und/oder optische Isomere bzw. Regioisomere oder deren Isomerengemische in unterschiedlicher Zusammensetzung vorliegen. Sowohl die reinen Isomere als auch die Isomerengemische werden erfindungsgemäß beansprucht.

Weiterhin wurde gefunden, dass sich Δ¹-Pyrroline der Formel (I) herstellen lassen, indem man A) Δ¹-Pyrroline der Formel (II) 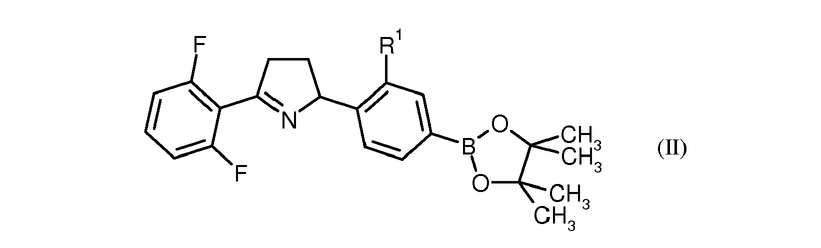 in welcher
- R¹: die oben angegebenen Bedeutungen hat,
mit Pyridinen der Formel (III) 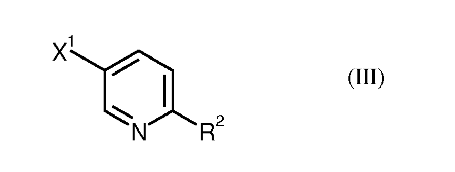 in welcher
- R²: die oben angegebenen Bedeutungen hat und
- X¹: für Brom, Chlor, oder Jod steht,
- X¹: bevorzugt für Brom oder Chlor steht,
in Gegenwart eines Katalysators und in Gegenwart eines Verdünnungsmittels umsetzt.

Schließlich wurde gefunden, dass die erfindungsgemäßen Verbindungen der Formel (I) sehr gute insektizide Eigenschaften besitzen und sich im Pflanzenschutz, im Materialschutz, sowie im veterinärmedizinischen Sektor zur Bekämpfung unerwünschter Schädlinge, wie Insekten, verwenden lassen.

Die erfindungsgemäßen Δ¹-Pyrroline sind durch die Formel (I) allgemein definiert.

Bevorzugte Substituenten bzw. Bereiche der in den oben und nachstehend erwähnten Formeln aufgeführten Reste werden im folgenden erläutert.
- R¹: steht bevorzugt für Chlor, Fluor oder Brom
- R²: steht bevorzugt für jeweils gegebenenfalls einfach oder mehrfach, gleich oder verschieden durch Halogen, oder Cyano substituiertes Alkyl, Alkenyl, Alkoxy, Alkenyloxy; oder für jeweils gegebenenfalls einfach oder mehrfach, gleich oder verschieden durch Halogen, Cyano, Nitro, Alkyl, Halogenalkyl, Alkenyl, Halogenalkenyl, Alkoxy, Halogenalkoxy, Alkylthio und/oder Halogenalkylthio substituiertes Cycloalkyl, Cycloalkoxy.
- R¹: steht besonders bevorzugt für Chlor oder Fluor
- R²: steht besonders bevorzugt für jeweils gegebenenfalls einfach oder mehrfach, gleich oder verschieden durch Halogen oder Cyano substituiertes C₁-C₅-Alkyl, C₁-C₅-Alkoxy; oder für jeweils gegebenenfalls einfach bis vierfach, gleich oder verschieden, durch Halogen, Cyano, Nitro, C₁-C₆-Alkyl, C₁-C₆-Halogenalkyl, C₂-C₆-Alkenyl, C₂-C₆-Halogenalkenyl, C₁-C₆-Alkoxy, C₁-C₆-Halogenalkoxy, C₁-C₆-Alkylthio, C₁-C₆-Halogenalkylthio substituiertes C₃-C₅-Cycloalkyl, C₃-C₅-Cycloalkoxy.
- R¹: steht ganz besonders bevorzugt für Chlor;
- R¹: steht weiterhin ganz besonders bevorzugt für Fluor
- R²: steht ganz besonders bevorzugt; für iso-propoxy, propoxy, butoxy, cyclobutoxy, pentoxy.
Weiterhin ganz besonders bevorzugt sind (R)-konfigurierte Verbindungen der Formeln (I-1) 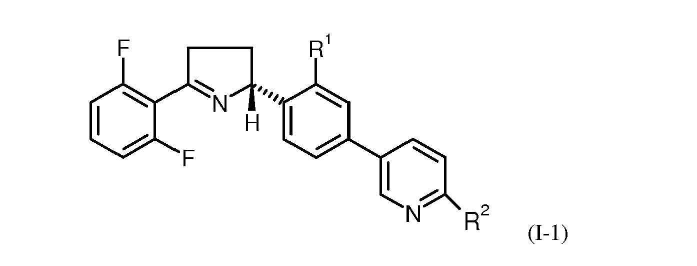 in welcher R¹ und R² die oben angegebenen, insbesondere die unter bevorzugt, besonders bevorzugt und ganz besonders bevorzugt angegebenen Bedeutungen haben.

Weiterhin ganz besonders bevorzugt sind Verbindungen der Formel (I-1), in welcher R¹ für Fluor steht.

Weiterhin ganz besonders bevorzugt sind Verbindungen der Formel (I-1), in welcher R¹ für Chlor steht.

Gesättigte Kohlenwasserstoffreste wie Alkyl können, auch in Verbindung mit Heteroatomen, wie z.B. in Alkoxy, soweit möglich, jeweils geradkettig oder verzweigt sein.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend.

Verwendet man R-Enantiomere von Δ¹-Pyrroline der Formel (II-1) und halogenierte Pyridine der Formel (III) als Ausgangsstoffe sowie einen Palladiumkatalysator, so lassen sich durch Suzuki-Kupplung die R-Enantiomere der erfindungsgemässen Wirkstoffe erhalten. Der Verlauf des erfindungsgemäßen Verfahrens (A) kann durch folgendes Formelschema veranschaulicht werden:

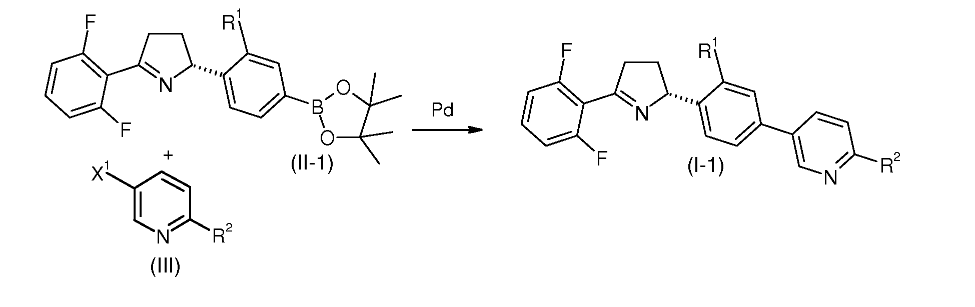

Die bei der Durchführung des erfindungsgemäßen Verfahrens (A) als Ausgangsstoffe benötigten Δ¹-Pyrroline sind durch die Formel (II-1) allgemein definiert. In dieser Formel steht R¹ bevorzugt, besonders bevorzugt, bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden.

Δ¹-Pyrroline der Formel (II) sind bekannt und/oder lassen sich nach bekannten Verfahren herstellen (vgl. WO 04/031176). Durch übliche Verfahren zur Racematspaltung, wie zum Beispiel durch Chromatographie der entsprechenden Racemate an einer chiralen stationären Phase sind die R-Enantiomere der Formel (II-1) zugänglich.

Die bei der Durchführung des erfindungsgemäßen Verfahrens (A) als Ausgangsstoffe benötigten Pyridine sind durch die Formel (III) allgemein definiert. In dieser Formel stehen X¹ und R² bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden.

Pyridine der Formel (III) sind teilweise bekannt und/oder können in analoger Weise zu bekannten Verbindungen herstellt werden (vgl. WO 04/031176, WO 05/035522, WO 96/15096, Gu, Yu Gui et. al, Journal of Medicinal Chemistry 2006, 49(13), 3770-3773).

Bei der Durchführung des erfindungsgemäßen Verfahrens (A) setzt man bevorzugt einen Palladium-Katalysator ein, der wiederum mit oder ohne Zusatz von weiteren Liganden, oder von Phasentransferkatalysatoren verwendet werden kann. Vorzugsweise verwendet man als Katalysator PdCl₂(dppf) [dppf = 1,1'-Bis(diphenylphosphino)ferrocene], Pd(PPh₃)₄, PdCl₂(PPh₃)₂, PdCl₂(CH₃CN)₂, Pd₂(dba)₃ [dba = Dibenzylidenaceton], Pd(NO₃)₂, Tetra-n-butylammoniumbromid oder Pd(OAc)₂, besonders bevorzugt Pd(OAc)₂, Pd(NO₃)₂, Tetra-n-butylammoniumbromid.

Als Liganden kommen Triarylphosphine, Trialkylphosphine oder Arsine in Frage. Vorzugsweise verwendet man dppf, PPh₃, P(tert-Bu)₃, Pcy₃ oder AsPh₃, besonders bevorzugt dppf.

Als Verdünnungsmittel kommen bei der Durchführung des erfindungsgemäßen Verfahrens (A) jeweils alle üblichen inerten, organischen Solventien oder Wasser in Frage. Vorzugsweise verwendbar sind gegebenenfalls halogenierte aliphatische, alicyclische oder aromatische Kohlenwasserstoffe, wie Petrolether, Hexan, Heptan, Cyclohexan, Methylcyclohexan, Benzol, Toluol, Xylol oder Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Nitrile, wie Acetonitril, Propionitril, n- oder iso-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid oder Sulfone, wie Sulfolan oder Wasser. Besonders bevorzugt verwendet man Aceton, Dimethoxyethan, Dioxan, Tetrahydrofuran, Dimethylformamid, Dimethylacetamid, Dimethylsulfoxid, Ethanol, Toluol oder gegebenenfalls Gemische dieser genannten Verdünnungsmittel mit Wasser oder Wasser alleine.

Als Base kommen bei der Durchführung der erfindungsgemäßen Verfahrens (A) alle für derartige Reaktionen üblichen anorganischen und organischen Basen in Betracht. Vorzugsweise verwendbar sind Erdalkali- oder Alkalimetallhydroxide, wie Natriumhydroxid, Calciumhydroxid, Kaliumhydroxid, oder auch Ammoniumhydroxid, Alkalimetallcarbonate, wie Natriumcarbonat, Kaliumcarbonat, Kaliumhydrogencarbonat, Natriumhydrogencarbonat, Alkali- oder Erdalkalimetallacetate wie Natriumacetat, Kaliumacetat, Calciumacetat, Alkalimetallfluoride, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU). Besonders bevorzugt verwendet man Bariumhydroxid, Natriumhydroxid, Kaliumhydroxid, Trikaliumphosphat, Caesiumcarbonat, Kaliumcarbonat, Natriumcarbonat, Kaliumacetat, Triethylamin, Kalium-tert-butanolat, Caesiumfluorid oder Kaliumfluorid.

Die Reaktionstemperaturen können bei der Durchführung des erfindungsgemäßen Verfahrens (A) in einem größeren Bereich variiert werden. Im allgemeinen arbeitet man bei Temperaturen zwischen 0°C und 140°C, vorzugsweise zwischen 20°C und 120°C, besonders bevorzugt zwischen 60°C und 100°C.

Bei der Durchführung des erfindungsgemäßen Verfahrens (A) setzt man auf 1 Mol an Verbindung der Formel (II-1) im allgemeinen 1 Mol oder einen leichten Überschuss einer Verbindung der Formel (III), sowie 0,1 bis 50 Mol% eines Katalysators ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch mit Wasser verdünnt und mit einem organischen Verdünnungsmittel extrahiert. Die organische Phase wird gewaschen, getrocknet, filtriert und eingeengt. Der Rückstand wird gegebenenfalls nach üblichen Methoden, wie Chromatographie oder Umkristallisation, von eventuell noch vorhandenen Verunreinigungen befreit.

Bei der Durchführung aller erfindungsgemäßen Verfahren arbeitet man im allgemeinen unter Atmosphärendruck. Es ist aber auch möglich, jeweils unter erhöhtem oder vermindertem Druck zu arbeiten.

Die erfindungsgemäßen Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spp.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Acheta domesticus, Gryllotalpa spp., Locusta migratoria migratorioides, Melanoplus spp., Schistocerca gregaria.
Aus der Ordnung der Blattaria z.B. Blatta orientalis, Periplaneta americana, Leucophaea maderae, Blattella germanica.
Aus der Ordnung der Dermaptera z.B. Forficula auricularia.
Aus der Ordnung der Isoptera z.B. Reticulitermes spp..
Aus der Ordnung der Phthiraptera z.B. Pediculus humanus corporis, Haematopinus spp., Linognathus spp., Trichodectes spp., Damalinia spp..
Aus der Ordnung der Thysanoptera z.B. Hercinothrips femoralis, Thrips tabaci, Thrips palmi, Frankliniella accidentalis.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp.,
Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..
Aus der Ordnung der Siphonaptera z.B. Xenopsylla cheopis, Ceratophyllus spp..
Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp..
Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp..
Die erfindungsgemäßen Verbindungen der Formel (I) zeichnen sich insbesondere durch eine hervorragende Wirkung gegen Raupen, Käferlarven, Spinnmilben, Blattläuse und Minierfliegen aus.
Die erfindungsgemäßen Verbindungen können gegebenenfalls in bestimmten Konzentrationen bzw. Aufwandmengen auch als Herbizide und Mikrobizide, beispielsweise als Fungizide, Antimykotika und Bakterizide verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die erfindungsgemäßen Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der erfindungsgemäßen Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln;
als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate;
als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

### Besonders günstige Mischpartner sind z.B. die folgenden:

### Fungizide:

Aldimorph, Ampropylfos, Ampropylfos-Kalium, Andoprim, Anilazin, Azaconazol, Azoxystrobin,

Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,

Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,

Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,

Ediphenphos, Epoxiconazol, Etaconazol, Ethirimol, Etridiazol,

Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox, Guazatin, Hexachlorobenzol, Hexaconazol, Hymexazol,

Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,

Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,

Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,

Nickel-dimethyldithiocarbamat, Nitrothal-isopropyl, Nuarimol,

Ofurace, Oxadixyl, Oxamocarb, Oxolinicacid, Oxycarboxim, Oxyfenthiin,

Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,

Quinconazol, Quintozen (PCNB),

Schwefel und Schwefel-Zubereitungen,

Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol, Uniconazol, Validamycin A, Vinclozolin, Viniconazol, Zarilamid, Zineb, Ziram sowie Dagger G, OK-8705, OK-8801, α-(1,1-Dimethylethyl)-β-(2-phenoxyethyl)-1H-1,2,4-triazol-1-ethanol, α-(2,4-Dichlorphenyl)-β-fluor-b-propyl-1H-1,2,4-triazol-1-ethanol, α-(2,4-Dichlorphenyl)-β-methoxy-a-methyl-1H-1,2,4-triazol-1-ethanol, α-(5-Methyl-1,3-dioxan-5-yl)-β-[[4-(trifluormethyl)-phenyl]-methylen]-1H-1,2,4-triazol-1-ethanol, (5RS,6RS)-6-Hydroxy-2,2,7,7-tetramethyl-5-(1H-1,2,4-triazol-1-yl)-3-octanon, (E)-a-(Methoxyimino)-N-methyl-2-phenoxy-phenylacetamid, {2-Methyl-1-[[[1-(4-methylphenyl)-ethyl]-amino]-carbonyl]-propyl}-carbaminsäure-1-isopropylester 1-(2,4-Dichlorphenyl)-2-(1H-1,2,4-triazol-1-yl)-ethanon-O-(phenylmethyl)-oxim, 1-(2-Methyl-1-naphthalenyl)-1H-pyrrol-2,5-dion, 1-(3,5-Dichlorphenyl)-3-(2-propenyl)-2,5-pyrrolidindion, 1-[(Diiodmethyl)-sulfonyl]-4-methyl-benzol, 1-[[2-(2,4-Dichlorphenyl)-1,3-dioxolan-2-yl]-methyl]-1H-imidazol, 1-[[2-(4-Chlorphenyl)-3-phenyloxiranyl]-methyl]-1H-1,2,4-triazol, 1-[1-[2-[(2,4-Dichlorphenyl)-methoxy]-phenyl]-ethenyl]-1H-imidazol, 1-Methyl-5-nonyl-2-(phenylmethyl)-3-pyrrolidinol, 2',6'-Dibrom-2-methyl-4'-trifluormethoxy-4'-trifluor-methyl-1,3-thiazol-5-carboxanilid, 2,2-Dichlor-N-[1-(4-chlorphenyl)-ethyl]-1-ethyl-3-methyl-cyclopropancarboxamid, 2,6-Dichlor-5-(methylthio)-4-pyrimidinyl-thiocyanat, 2,6-Dichlor-N-(4-trifluormethylbenzyl)-benzamid, 2,6-Dichlor-N-[[4-(trifluormethyl)-phenyl]-methyl]-benzamid, 2-(2,3,3-Triiod-2-propenyl)-2H-tetrazol, 2-[(1-Methylethyl)-sulfonyl]-5-(trichlormethyl)-1,3,4-thiadiazol, 2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril, 2-Aminobutan, 2-Brom-2-(brommethyl)-pentandinitril, 2-Chlor-N-(2,3-dihydro-1,1,3-trimethyl-1H-inden-4-yl)-3-pyridincarboxamid, 2-Chlor-N-(2,6-dimethylphenyl)-N-(isothiocyanatomethyl)-acetamid, 2-Phenylphenol(OPP), 3,4-Dichlor-1-[4-(difluormethoxy)-phenyl]-1H-pyrrol-2,5-dion, 3,5-Dichlor-N-[cyan[(1-methyl-2-propynyl)-oxy]-methyl]-benzamid, 3-(1,1-Dimethylpropyl-1-oxo-1H-inden-2-carbonitril, 3-[2-(4-Chlorphenyl)-5-ethoxy-3-isoxazolidinyl]-pyridin, 4-Chlor-2-cyan-N,N-dimethyl-5-(4-methylphenyl)-1H-imidazol-1-sulfonamid, 4-Methyl-tetrazolo[1,5-a]quinazolin-5(4H)-on, 8-(1,1-Dimethylethyl)-N-ethyl-N-propyl-1,4-dioxaspiro[4.5]decan-2-methanamin, 8-Hydroxychinolinsulfat, 9H-Xanthen-9-carbonsäure-2-[(phenylamino)-carbonyl]-hydrazid, bis-(1-Methylethyl)-3-methyl-4-[(3-methylbenzoyl)-oxy]-2,5-thiophendicarboxylat, cis-1-(4-Chlorphenyl)-2-(1H-1,2,4-triazol-1-yl)-cycloheptanol, cis-4-[3-[4-(1,1-Dimethylpropyl)-phenyl-2-methylpropyl]-2,6-dimethyl-morpholin-hydrochlorid, Ethyl-[(4-chlorphenyl)-azo]-cyanoacetat, Kaliumhydrogencarbonat, Methantetrathiol-Natriumsalz, Methyl-1-(2,3-dihydro-2,2-dimethyl-1H-inden-1-yl)-1H-imidazol-5-carboxylat, Methyl-N-(2,6-dimethylphenyl)-N-(5-isoxazolylcarbonyl)-DL-alaninat, Methyl-N-(chloracetyl)-N-(2,6-dimethylphenyl)-DL-alaninat, N-(2,3-Dichlor-4-hydroxyphenyl)-1-methyl-cyclohexancarboxamid. N-(2,6-Dimethylphenyl)-2-methoxy-N-(tetrahydro-2-oxo-3-furanyl)-acetamid, N-(2,6-Dimethylphenyl)-2-methoxy-N-(tetrahydro-2-oxo-3-thienyl)-acetamid, N-(2-Chlor-4-nitrophenyl)-4-methyl-3-nitro-benzolsulfonamid, N-(4-Cyclohexylphenyl)-1,4,5,6-tetrahydro-2-pyrimidinamin, N-(4-Hexylphenyl)-1,4,5,6-tetrahydro-2-pyrimidinamin, N-(5-Chlor-2-methylphenyl)-2-methoxy-N-(2-oxo-3-oxazolidinyl)-acetamid, N-(6-Methoxy)-3-pyridinyl)-cyclopropancarboxamid, N-[2,2,2-Trichlor-1-[(chloracetyl)-amino]-ethyl]-benzamid, N-[3-Chlor-4,5-bis-(2-propinyloxy)-phenyl]-N'-methoxy-methanimidamid, N-Formyl-N-hydroxy-DL-alanin -Natriumsalz, O,O-Diethyl-[2-(dipropylamino)-2-oxoethyl]-ethylphosphoramidothioat, O-Methyl-S-phenyl-phenylpropylphosphoramidothioat, S-Methyl-1,2,3-benzothiadiazol-7-carbothioat, spiro[2H]-1-Benzopyran-2,1'(3'H)-isobenzofuran]-3'-on,

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Acetylcholinesterase (AChE) Inhibitoren II-1

### II-1.A Carbamate,

zum Beispiel Alanycarb (II-1.A-1), Aldicarb (II-1.A-2), Aldoxycarb (II-1.A-3), Allyxycarb (II-1.A-4), Aminocarb (II-1.A-5), Bendiocarb (II-1.A-6), Benfuracarb (II-1.A-7), Bufencarb (II-1.A-8), Butacarb (II-1.A-9), Butocarboxim (11-1.A-10), Butoxycarboxim (II-1.A-11), Carbaryl (II-1.A-12), Carbofuran (II-1.A-13), Carbosulfan (II-1.A-14), Cloethocarb (II-1.A-15), Dimetilan (II-1.A-16), Ethiofencarb (II-1.A-17), Fenobucarb (II-1.A-18), Fenothiocarb (II-1.A-19), Formetanate (II-1.A-20), Furathiocarb (II-1.A-21), Isoprocarb (II-1.A-22), Metam-sodium (II-1.A-23), Methiocarb (II-1.A-24), Methomyl (II-1.A-25), Metolcarb (II-1.A-26), Oxamyl (II-1.A-27), Pirimicarb (II-1.A-28), Promecarb (II-1.A-29), Propoxur (II-1.A-30), Thiodicarb (II-1.A-31), Thiofanox (II-1.A-32), Trimethacarb (II-1.A-33), XMC (II-1.A-34), Xylylcarb (II-1.A-35)

### II-1.B Organophosphate,

zum Beispiel Acephate (II-1.B-1), Azamethiphos (II-1.B-2), Azinphos (-methyl, -ethyl) (II-1.B-3), Bromophos-ethyl (II-1.B-4), Bromfenvinfos (-methyl) (II-1.B-5), Butathiofos (II-1.B-6), Cadusafos (II-1.B-7), Carbophenothion (II-1.B-8), Chlorethoxyfos (II-1.B-9), Chlorfenvinphos (II-1.B-10), Chlormephos (II-1.B-11), Chlorpyrifos (-methyl/-ethyl) (II-1.B-12), Coumaphos (II-1.B-13), Cyanofenphos (II-1.B-14), Cyanophos (II-1.B-15), Chlorfenvinphos (II-1.B-16), Demeton-S-methyl (II-1.B-17), Demeton-S-methylsulphon (II-1.B-18), Dialifos (II-1.B-19), Diazinon (II-1.B-20), Dichlofenthion (II-1.B-21), Dichlorvos/DDVP (II-1.B-22), Dicrotophos (II-1.B-23), Dimethoate (II-1.B-24), Dimethylvinphos (II-1.B-25), Dioxabenzofos (II-1.B-26), Disulfoton (II-1.B-27), EPN (II-1.B-28), Ethion (II-1.B-29), Ethoprophos (II-1.B-30), Etrimfos (II-1.B-31), Famphur (II-1.B-32), Fenamiphos (II-1.B-33), Fenitrothion (II-1.B-34), Fensulfothion (II-1.B-35), Fenthion (II-1.B-36), Flupyrazofos (II-1.B-37), Fonofos (II-1.B-38), Formothion (II-1.B-39), Fosmethilan (II-1.B-40), Fosthiazate (II-1.B-41), Heptenophos (II-1.B-42), Iodofenphos (II-1.B-43), Iprobenfos (II-1.B-44), Isazofos (II-1.B-45), Isofenphos (II-1.B-46), Isopropyl (II-1.B-47), O-salicylate (II-1.B-48), Isoxathion (II-1.B-49), Malathion (II-1.B-50), Mecarbam (II-1.B-51), Methacrifos (II-1.B-52), Methamidophos (II-1.B-53), Methidathion (II-1.B-54), Mevinphos (II-1.B-55), Monocrotophos (II-1.B-56), Naled (II-1.B-57), Omethoate (II-1.B-58), Oxydemeton-methyl (II-1.B-59), Parathion (-methyl/-ethyl) (II-1.B-60), Phenthoate (II-1.B-61), Phorate (II-1.B-62), Phosalone (II-1.B-63), Phosmet (II-1.B-64), Phosphamidon (II-1.B-65), Phosphocarb (II-1.B-66), Phoxim (II-1.B-67), Pirimiphos (-methyl/-ethyl) (II-1.B-68), Profenofos (II-1.B-69), Propaphos (II-1.B-70), Propetamphos (II-1.B-71), Prothiofos (II-1.B-72), Prothoate (II-1.B-73), Pyraclofos (II-1.B-74), Pyridaphenthion (II-1.B-75), Pyridathion (II-1.B-76), Quinalphos (II-1.B-77), Sebufos (II-1.B-78), Sulfotep (II-1.B-79), Sulprofos (II-1.B-80), Tebupirimfos (II-1.B-81), Temephos (II-1.B-82), Terbufos (II-1.B-83), Tetrachlorvinphos (II-1.B-84), Thiometon (II-1.B-85), Triazophos (II-1.B-86), Triclorfon (II-1.B-87), Vamidothion (II-1.B-88)
GABA-gesteuerte Chlorid-Kanal-Antagonisten II-2

### II-2A Organochlorine,

zum Beispiel Camphechlor (II-2A-1), Chlordane (II-2A-2), Endosulfan (II-2A-3), Gamma-HCH (II-2A-4), HCH (II-2A-5), Heptachlor (II-2A-6), Lindane (II-2A-7), Methoxychlor (II-2A-8)

### II-2B Fiprole (Phenylpyrazole),

zum Beispiel Acetoprole (II-2B-1), Ethiprole (II-2B-2), Fipronil (II-2B-3), Pyrafluprole (II-2B-4), Pyriprole (II-2B-5), Vaniliprole (II-2B-6)

Natrium-Kanal-Modulatoren / Spannungsabhängige Natrium-Kanal-Blocker II-3

### II-3 Pyrethroide,

zum Beispiel Acrinathrin (11-3-1), Allethrin (d-cis-trans, d-trans) (11-3-2), Beta-Cyfluthrin (11-3-3), Bifenthrin (11-3-4), Bioallethrin (11-3-5), Bioallethrin-S-cyclopentyl-isomer (11-3-6), Bioethanomethrin (11-3-7), Biopermethrin (11-3-8), Bioresmethrin (11-3-9), Chlovaporthrin (11-3-10), Cis-Cypermethrin (11-3-11), Cis-Resmethrin (11-3-12), Cis-Permethrin (11-3-13), Clocythrin (11-3-14), Cycloprothrin (11-3-15), Cyfluthrin (11-3-16), Cyhalothrin (II-3-17), Cypermethrin (alpha-, beta-, theta-, zeta-)(II-3-18), Cyphenothrin (II-3-19), Deltamethrin (11-3-20), Empenthrin (1R-isomer) (11-3-21), Esfenvalerate (11-3-22), Etofenprox (11-3-23), Fenfluthrin (11-3-24), Fenpropathrin (11-3-25), Fenpyrithrin (11-3-26), Fenvalerate (11-3-27), Flubrocythrinate (11-3-28), Flucythrinate (11-3-29), Flufenprox (11-3-30), Flumethrin (II-3-31), Fluvalinate (11-3-32), Fubfenprox (11-3-33), Gamma-Cyhalothrin (11-3-34), Imiprothrin (11-3-35), Kadethrin (11-3-36), Lambda-Cyhalothrin (11-3-37), Metofluthrin (11-3-38), Permethrin (cis-, trans-) (11-3-39), Phenothrin (1R-trans isomer) (II-3-40), Prallethrin (11-3-41), Profluthrin (11-3-42), Protrifenbute (11-3-43), Pyresmethrin (II-3-44), Resmethrin (11-3-45), RU 15525 (11-3-46), Silafluofen (11-3-47), Tau-Fluvalinate (II-3-48), Tefluthrin (11-3-49), Terallethrin (11-3-50), Tetramethrin (-1R- isomer) (11-3-51), Tralomethrin (11-3-52), Transfluthrin (11-3-53), ZXI 8901 (11-3-54), Pyrethrin (pyrethrum) (11-3-55), Eflusilanat (11-3-56), DDT (11-3-57), Methoxychlor (11-3-58),
Nikotinerge Acetylcholin-Rezeptor-Agonisten/-Antagonisten II-4

### II-4A Chloronicotinyle,

zum Beispiel Acetamiprid (II-4A-1), Clothianidin (II-4A-2), Dinotefuran (II-4A-3), Imidacloprid (II-4A-4), Imidaclothiz (II-4A-5), Nitenpyram (II-4A-6), Nithiazine (II-4A-7), Thiacloprid (II-4A-8), Thiamethoxam (II-4A-9),

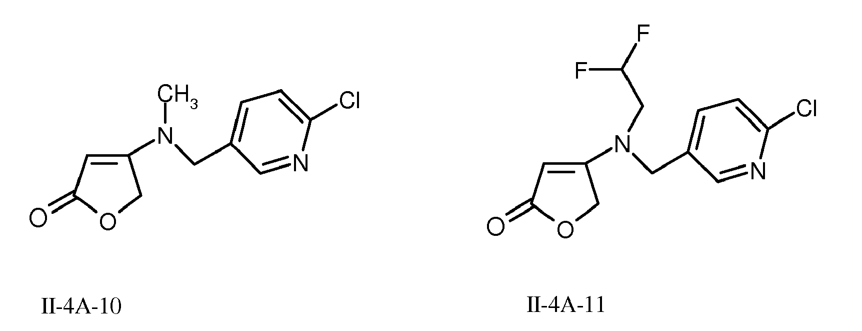

### II-4B Nicotine (II-4B-1), Bensultap (II-4B-2), Cartap (II-4B-3), Thiosulfap-Natrium (II-4B-4), Thiocylam (II-4C-4)

Allosterische Acetylcholin-Rezeptor-Modulatoren (Agonisten)

### II-5 Spinosyne,

zum Beispiel Spinosad (II-5-1), Spinetoram (II-5-2) Chlorid-Kanal-Aktivatoren

### II-6 Mectine / Macrolide,

zum Beispiel Abamectin (II-6-1), Emamectin (11-6-2), Emamectin-benzoate (11-6-3), Ivermectin (11-6-4), Lepimectin (11-6-5), Milbemectin (II-6-6)

### II-7A Juvenilhormon Analoge,

zum Beispiel Hydroprene (II-7A-1), Kinoprene (II-7A-2), Methoprene (II-7A-3), Epofenonane (II-7A-4), Triprene (II-7A-5), Fenoxycarb (II-7B-1), Pyriproxifen (II-7C-1), Diofenolan (II-7C-2) Wirkstoffe mit unbekannten oder nicht spezifischen Wirkmechanismen

### II-8 Begasungsmittel,

zum Beispiel Methyl bromide (II-8A-1), Chloropicrin (II-8B-1), Sulfuryl fluoride (II-8C-1)

### II-9 Selektive Fraßhemmer,

zum Beispiel Cryolite (II-9A-1), Pymetrozine (II-9B-1), NNI0101 (II-9B-2), Flonicamid (II-9C-1)

### II-10 Milbenwachstumsinhibitoren,

zum Beispiel Clofentezine (II-10A-1), Hexythiazox (II-10A-2),), Etoxazole (II-10B-1) Inhibitoren der oxidativen Phosphorylierung, ATP-Disruptoren II-12

### II-12A Diafenthiuron (II-12A-1)

### II-12B Organozinnverbindungen,

zum Beispiel Azocyclotin (II-12B-1), Cyhexatin (II-12B-2), Fenbutatin-oxide (II-12B-3)

### II-12C Propargite (II-12C-1), Tetradifon (II-12C-2)

Entkoppler der oxidativen Phoshorylierung durch Unterbrechung des H-Protongradienten 11-13 Chlorfenapyr (II-13-1) Binapacyrl (II-13-2), Dinobuton (II-13-3), Dinocap (II-13-4), DNOC (II-13-5) Mikrobielle Disruptoren der Insektendarmmembran Bacillus thuringiensis-Stämme (II-13-6) Inhibitoren der Chitinbiosynthese

### II-15 Benzoylharnstoffe,

zum Beispiel Bistrifluron (11-15-1), Chlorfluazuron (11-15-2), Diflubenzuron (11-15-3), Fluazuron (II-15-4), Flucycloxuron (II-15-5), Flufenoxuron (II-15-6), Hexaflumuron (11-15-7), Lufenuron (11-15-8), Novaluron (11-15-9), Noviflumuron (11-15-10), Penfluron (11-15-11), Teflubenzuron (II-15-12), Triflumuron (II-15-13)

### II-16 Buprofezin (II-16-1)

Häutungsstörende Wirkstoffe Cyromazine (II-17-1) Ecdysonagonisten/disruptoren (II-18)

### II-18A Diacylhydrazine,

zum Beispiel Chromafenozide (II-18A-1), Halofenozide (II-18A-2), Methoxyfenozide (II-18A-3), Tebufenozide (II-18A-4), JS-118 (II-18A-5) Azadirachtin (II-18B-1) Oktopaminerge Agonisten zum Beispiel Amitraz (II-19-1)

II-20 Seite-III-Elektronentransportinhibitoren/Seite-II-Elektronentransportinhibitoren Hydramethylnon (II-20A-1) Acequinocyl (II-20B-1) Fluacrypyrim (II-20C-1) Cyflumetofen (II-20D-1), Cyenopyrafen (II-20D-2) Elektronentransportinhibitoren

### II-21 Seite-I-Elektronentransportinhibitoren

aus der Gruppe der METI-Akarizide, zum Beispiel Fenazaquin (II-21-1), Fenpyroximate (II-21-2), Pyrimidifen (II-21-3), Pyridaben (II-21-4), Tebufenpyrad (II-21-5), Tolfenpyrad (II-21-6), Rotenone (II-21-7)

### II-22 Spannungsabhängige Natriumkanal-Blocker

zum Beispiel Indoxacarb (II-22A-1) zum Beispiel Metaflumizone (BAS 3201) (II-22B-1)

### II-23 Inhibitoren der Fettsäurebiosynthese

### II-23A Tetronsäure-Derivate

zum Beispiel Spirodiclofen (II-23A-1), Spiromesifen (II-23A-2)

### II-23B Tetramsäure-Derivate,

zum Beispiel Spirotetramat (II-23B-1)

### II-25 Neuronale Inhibitoren mit unbekannten Wirkmechanismus

Bifenazate (II-25-1) Ryanodinrezeptor-Effektoren

### II-28 Diamide,

zum Beispiel Flubendiamide (II-28-1), 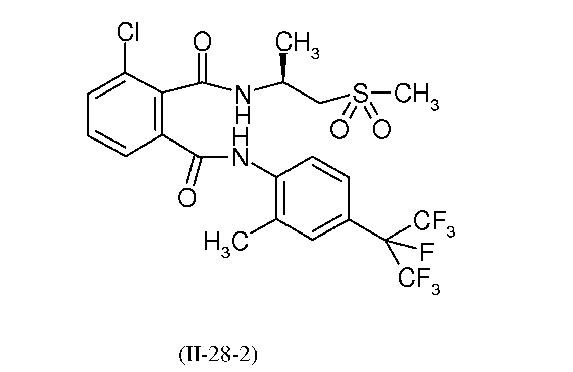 Rynaxapyr (II-28-3), Cyazypyr (HGW86)

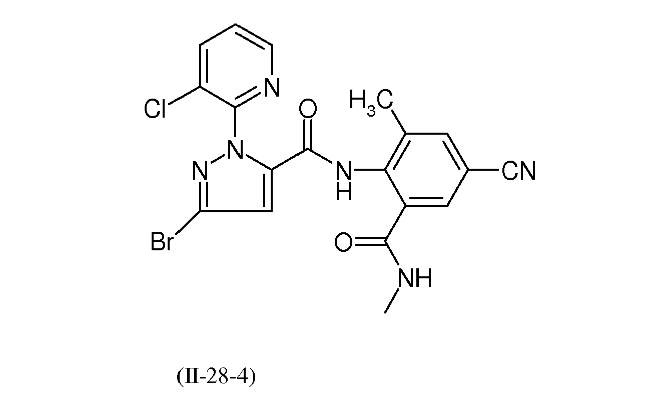

### II-29 Wirkstoffe mit unbekanntem Wirkmechanismis

Amidoflumet (11-29-1), Benclothiaz (11-29-2), Benzoximate (11-29-3), Bromopropylate (II-29-4), Buprofezin (11-29-5), Chinomethionat (11-29-6), Chlordimeform (11-29-7), Chlorobenzilate (11-29-8), Clothiazoben (11-29-9), Cycloprene (11-29-10), Dicofol (II-29-11), Dicyclanil (11-29-12), Fenoxacrim (11-29-13), Fentrifanil (11-29-14), Flubenzimine (11-29-15), Flufenerim (11-29-16), Flutenzin (11-29-17), Gossyplure (11-29-18), Japonilure (11-29-19), Metoxadiazone (11-29-20), Petroleum (II-29-21), Potassium oleate (11-29-22), Pyridalyl (II-29-23), Sulfluramid (11-29-24), Tetrasul (11-29-25), Triarathene (II-29-26),Verbutin (11-29-27).

Die in dieser Beschreibung mit ihrem "common name" genannten Wirkstoffe sind beispielsweise aus "The Pesticide Manual" 13th Ed., British Crop Protection Council 2003, und der Webseite http://\vww.alanwood.net/pesticides/ bekannt.

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der erfindungsgemäßen Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt.

Je nach Pflanzenarten bzw. Pflanzensorten, deren Standort und Wachstumsbedingungen (Böden, Klima, Vegetationsperiode, Ernährung) können durch die erfindungsgemäße Behandlung auch über additive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD^{®} (z.B. Mais, Baumwolle, Soja), KnockOut^{®} (z.B. Mais), StarLink^{®} (z.B. Mais), Bollgard^{®} (Baumwolle), Nucotn^{®} (Baumwolle) und NewLeaf^{®} (Kartoffel) vertrieben werden. Als Beispiele für Herbizid tolerante Pflanzen seien Maissorten, Baumwollsorten und Sojasorten genannt, die unter den Handelsbezeichnungen Roundup Ready^{®} (Toleranz gegen Glyphosate z.B. Mais, Baumwolle, Soja), Liberty Link^{®} (Toleranz gegen Phosphinotricin, z.B. Raps), IMI^{®} (Toleranz gegen Imidazolinone) und STS^{®} (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield^{®} vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören: Aus der Ordnung der Anoplurida z.B. Haematopinus spp., Linognathus spp., Pediculus spp., Phtirus spp., Solenopotes spp.. Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.. Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.. Aus der Ordnung der Siphonapterida z.B. Pulex spp., Ctenocephalides spp., Xenopsylla spp., Ceratophyllus spp.. Aus der Ordnung der Heteropterida z.B. Cimex spp., Triatoma spp., Rhodnius spp., Panstrongylus spp.. Aus der Ordnung der Blattarida z.B. Blatta orientalis, Periplaneta americana, Blattela germanica, Supella spp.. Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp.. Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine hervorragende Wirksamkeit gegen die Entwicklungsstadien von Zecken wie zum Beispiel Amblyomma hebraeum, gegen parasitierende Fliegen wie zum Beispiel gegen Lucilia cuprina, gegen Flöhe wie zum Beispiel Ctenocephalides felis

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die erfindungsgemäßen Wirkstoffe der Formel (I) als Formulierungen (beispielsweise Pulver, Emulsionen, fließfähige Mittel), die die erfindungsgemäßen Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:

### Käfer wie

Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

### Hautflügler wie

Sirex juvencus, Urocerus gigas, Urocerus gigas taignus, Urocerus augur.

### Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die erfindungsgemäßen Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der erfindungsgemäßen Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindelöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällen vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid und Triflumuron, sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butyl-carbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflusskrebse) zusammengefasst werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis(trialkylzinn)-sulfiden, Tri-n-butylzinnlaurat, Tri-n-butylzinnchlorid, Kupfer(I)-oxid, Triethylzinnchlorid, Tri-n-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-n-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:

### Algizide wie

2-*tert*.-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn; Fungizide wie

Benzo[*b*]thiophencarbonsäurecyclohexylamid-S,S-dioxid, Dichlofluanid, Fluorfolpet, 3-Iod-2-propinyl-butylcarbamat, Tolylfluanid und Azole wie

Azaconazole, Cyproconazole, Epoxyconazole, Hexaconazole, Metconazole, Propiconazole und Tebuconazole; Molluskizide wie Fentinacetat, Metaldehyd, Methiocarb, Niclosamid, Thiodicarb und Trimethacarb; oder herkömmliche Antifouling-Wirkstoffe wie 4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten des weiteren die üblichen Bestandteile wie z.B. in Ungerer, Chem. Ind. 1985, 37, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wässrigen System, Vinylchlorid/Vinylacetat-Copolymersysteme in Form wässriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die erfindungsgemäßen Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.

Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die Herstellung und die Verwendung der erfindungsgemäßen Stoffe geht aus den folgenden Beispielen hervor.

### Herstellunssbeispiele

### Beispiel 1-1-1:

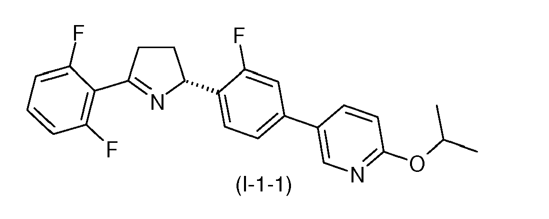

Zu einer Lösung von 5.0 g (R)-2-[2-Fluoro-4-(4,4,5,5-tetramethyl-1,3,2-dioxaborolan-2-yl)-phenyl]-5-(2,6-difluoro-phenyl)-3,4-dihydro-2H-pyrrol (Verbindung 11-1-1) in 200 ml Dimethoxyethan gibt man unter Argon nacheinander 2.9g 5-Bromo-2-isopropoxy-pyridin (Verbindung III-1-1), 18,7 ml einer 2molaren Natriumcarbonat-lösung und 0,28 g 1,1'-Bis(diphenylphosphino)-ferrocenpalladium(II)chlorid. Das Gemisch wird für 16 Stunden bei 80°C gerührt. Nach Aufarbeitung chromatographiert man an Kieselgel mit Dichloromethan/Essigsäureethylester im Verhältnis 10/1 und erhält so 4.2 g 5-{4-[5-(2,6-Difluoro-phenyl)-3,4-dihydro-2*H-*pyrrol-2-yl]-3-fluoro-phenyl}-2-isopropoxy-pyridin. 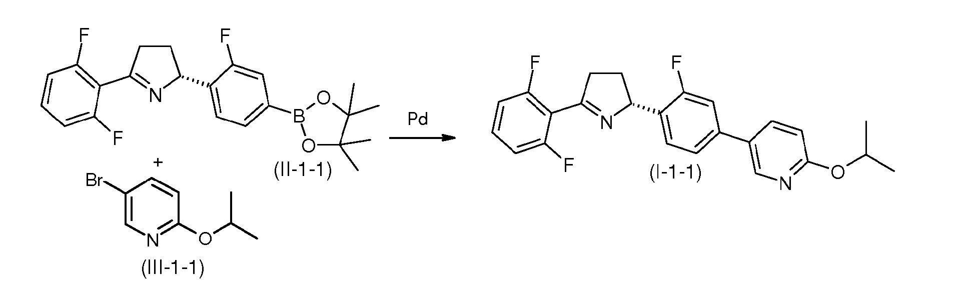 Charakterisierung der Verbindung I-1-1: ¹H-NMR (400 MHz, CD₃CN) δ = 1.33 (d, 6 H), 1.81 (m, 1 H), 2.62 (m, 1 H), 3.05 (m, 2 H), 5.31 (septett, 1 H), 5.51 (m, 1 H), 6.73 (d, 1 H), 7.07 (m, 2 H), 7.37 - 7.50 (m, 4 H), 7.85 (m, 1 H), 8.38 (m, 1 H).

LC-MS (Zorbax Eclipse, Ameisensäure/Acetonitril, ESI Positiv): m/z = M+H 411 Analog zu Beispiel I-1-1 werden durch Umsetzung der Verbindung (II-1-1) mit den entsprechenden Verbindungen der Formel (III-1) gemäß der Tabelle 1

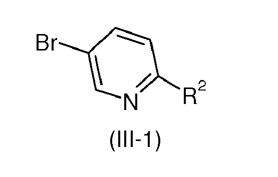

**Tabelle 1:**

| Bsp_{.} Nr. | R² |
|---|---|
| III-1-1 | isopropoxy |
| III-1-2 | propoxy |
| III-1-3 | butoxy |
| III-1-4 | cyclobutoxy |
| III-1-5 | pentoxy |

die Beispiele 1-1-2 bis 1-1-5 erhalten.

### Beispiel I-1-2:

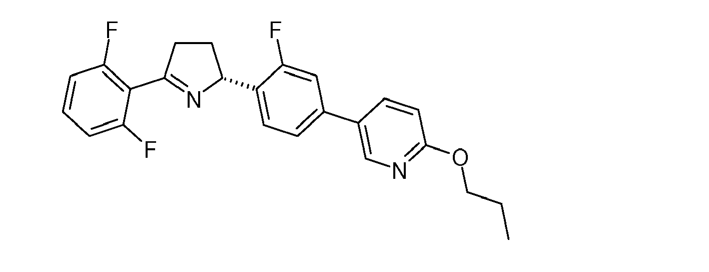

¹H-NMR (400 MHz, CD₃CN) δ = 1.01 (t, 3 H), 1.78 (m, 2 H), 1,88 (m, 1 H), 2.64 (m, 1 H), 3.07 (m, 2 H), 4.29 (t, 2 H), 5.54 (m, 1 H), 6.80 (d, 1 H), 7.07 (m, 2 H), 7.35 - 7.48 (m, 4 H), 7.88 (m, 1 H), 8.40(m, 1H). LC-MS (Zorbax Eclipse, Ameisensäure/Acetonitril, ESI Positiv): m/z = M+H 411

### Beispiel 1-1-3:

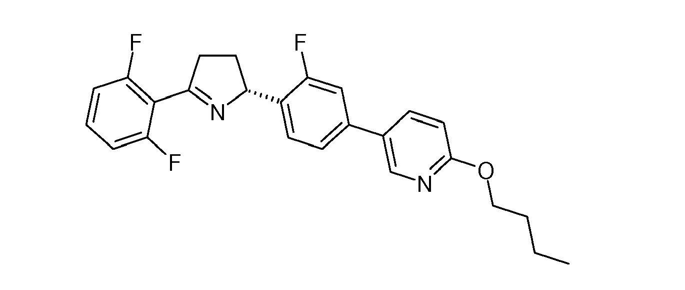

¹H-NMR (400 MHz, CD₃CN) δ = 0.97 (t, 3 H), 1.47 (m, 2 H), 1.72 (m, 2 H), 1.84 (m, 1 H), 2.63 (m, 1 H), 3.09 (m, 2 H), 4.32 (t, 2 H), 5.56 (m, 1 H), 6.82 (d, 1 H), 7.09 (m, 2 H), 7.41 (m, 3 H), 7.48 (m, 1 H), 7.90 (m, 1 H), 8.40 (m, 1 H).

LC-MS (Zorbax Eclipse, Ameisensäure/Acetonitril, ESI Positiv): m/z = M+H 425

### Beispiel 1-1-4:

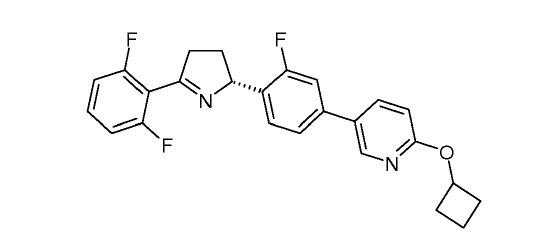

¹H-NMR (400 MHz, CD₃CN) δ = 1.65 - 2.15 (m, 3 H), 2.15 (m, 2 H), 2.45 (m, 2 H), 2.65 (m, 1 H), 3.07 (m, 2 H), 5.22 (m, 1 H), 5.54 (m, 1 H), 6.77 (d, 1 H), 7.06 (m, 2 H), 7.34 - 7.46 (m, 4 H), 7.88 (m, 1 H), 8.38 (m, 1 H). LC-MS (Kromasil , Ameisensäure/Acetonitril, ESI Positiv): m/z = M+H 423 **Beispiel 1-1-5:** 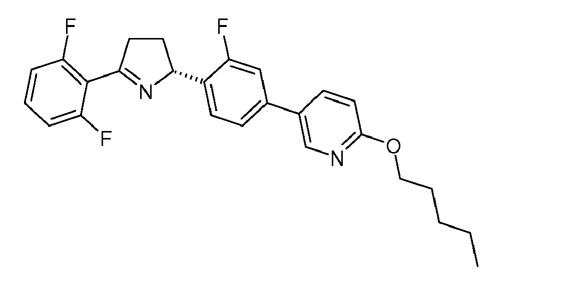 ¹H-NMR (400 MHz, CD₃CN) δ = 0.93 (t, 3 H), 1.41 (m, 4 H), 1.76 (m, 2 H), 1.86 (m, 1 H), 2.64 (m, 1 H), 3.08 (m, 2 H), 4.31 (t, 2 H), 5.33 (m, 1 H), 6.81 (d, 1 H), 7.09 (m, 2 H), 7.38- 7.50 (m, 3 H), 7.91 (m, 1 H), 8.41 (m, 1 H). LC-MS (Zorbax Eclipse, Ameisensäure/Acetonitril, ESI Positiv): m/z = M+H 439 In analoger Weise zu den Beispielen (I-1-1) bis (I-1-5) werden durch Umsetzung der Verbindung (II-1-2) 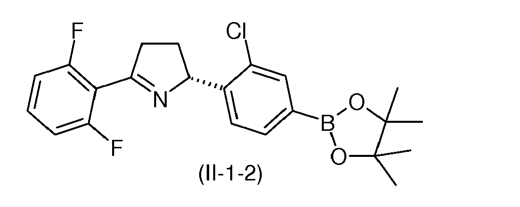 mit den entsprechenden Verbindungen der Formel (III-1) gemäß der Tabelle 1 die Beispiele I-1-6 bis I-1-10 erhalten:

### Beispiel 1-1-6:

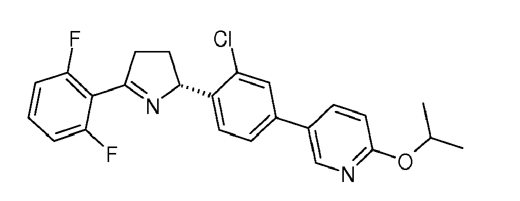

¹H-NMR (400 MHz, CD₃CN) δ = 1.33 (d, 6 H), 1.76 (m, 1 H), 2.74 (m, 1 H), 3.08 (m, 2 H), 5.33 (m, 1 H), 5.64 (m, 1 H), 6.75 (d, 1 H), 7.08 (m, 2 H), 7.38 (d, 1 H), 7.47 (m, 1 H), 7.53 (dd, 1 H), 7.68 (s, 1 H), 7.88 (dd, 1 H), 8.39 (d, 1 H). LC-MS (Kromasil , Ameisensäure/Acetonitril, ESI Positiv): m/z = M+H 427/429

### Beispiel 1-1-7:

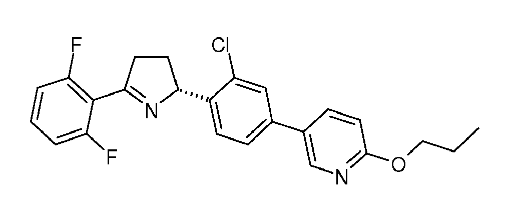

¹H-NMR (400 MHz, CD₃CN) δ = 1.01 (t, 3 H), 1.77 (m, 3 H), 2.75 (m, 1 H), 3.07 (m, 2 H), 4.29 (t, 2 H), 5.65 (m, 1 H), 6.81 (d, 1 H), 7.08 (m, 2 H), 7.38 (d, 1 H), 7.47 (m, 1 H), 7.53 (dd, 1 H), 7.68 (s, 1 H), 7.88 (dd, 1 H), 8.39 (d, 1 H). LC-MS (Kromasil , Ameisensäure/Acetonitril, ESI Positiv): m/z = M+H 427/429

### Beispiel 1-1-8:

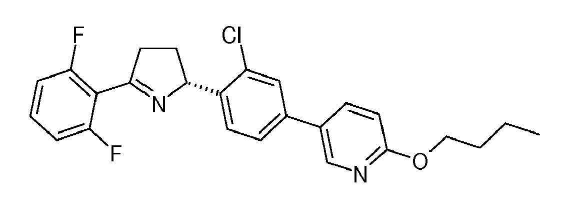

¹H-NMR (400 MHz, CD₃CN) δ = 0,97 (t, 3 H), 1,48 (m, 2 H), 1.75 (m, 3 H), 2.75 (m, 1 H), 3.06 (m, 2 H), 4,34 (m, 2 H), 5.64 (m, 1 H), 6.80 (d, 1 H), 7.08 (m, 2 H), 7.38 (d, 1 H), 7.47 (m, 1 H), 7.53 (dd, 1 H), 7.68 (s, 1 H), 7.88 (dd, 1 H), 8.40 (d, 1 H). LC-MS (Kromasil , Ameisensäure/Acetonitril, ESI Positiv): m/z = M+H 441/443

### Beispiel 1-1-9:

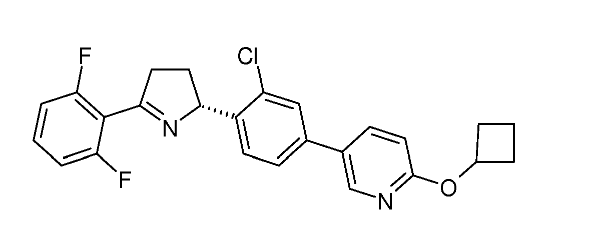

¹H-NMR (400 MHz, CD₃CN) δ = 1.72 (m, 2 H), 1,82 (m, 1 H), 2.13 (m, 2 H), 2.44 (m, 2 H), 2.76 (m, 1 H), 3.08 (m, 2 H), 5.22 (m, 1 H), 5.63 (m, 1 H), 6.79 (d, 1 H), 7.11 (m, 2 H), 7.38 (d, 1 H), 7.48 (m, 1 H), 7.55 (dd, 1 H), 7.71 (s, 1 H), 7.90 (dd, 1 H), 8.39 (d, 1 H). LC-MS (Kromasil , Ameisensäure/Acetonitril, ESI Positiv): m/z = M+H 439/441

### Beispiel 1-1-10:

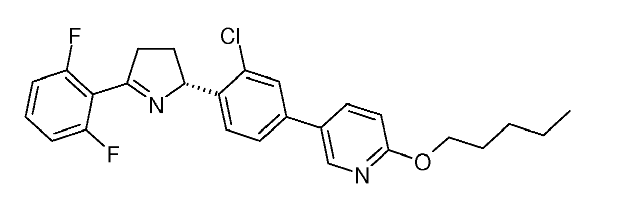

¹H-NMR (400 MHz, CD₃CN) δ = 0,93 (t, 3 H), 1,38 (m, 4 H), 1.74 (m, 3 H), 2.79 (m, 1 H), 3.08 (m, 2 H), 4,32 (m, 2 H), 5.64 (m, 1 H), 6.81 (d, 1 H), 7.10 (m, 2 H), 7.39 (d, 1 H), 7.50 (m, 1 H), 7.56 (dd, 1 H), 7.71 (s, 1 H), 7.92 (dd, 1 H), 8.40 (d, 1 H). LC-MS (Zorbax Eclipse, Ameisensäure/Acetonitril, ESI Positiv): m/z = M+H 455/457 Analog können nach dem Verfahren (A) die in der folgenden Tabelle genannten Verbindungen (I-1-11) bis (I-1-32) hergestellt werden.

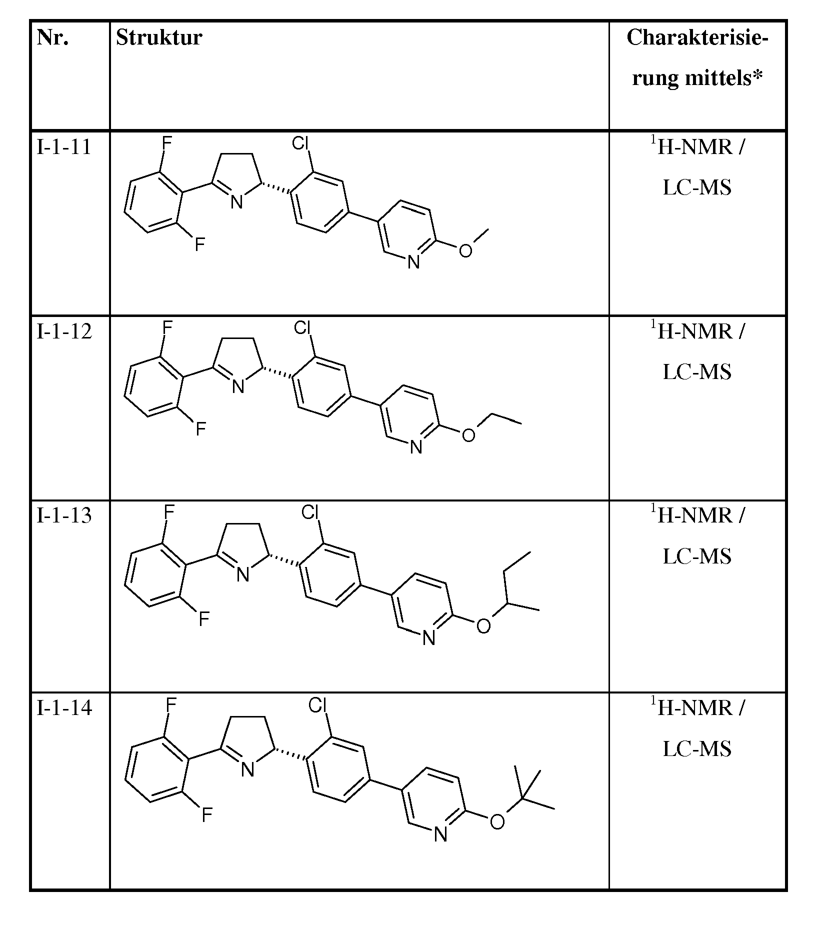

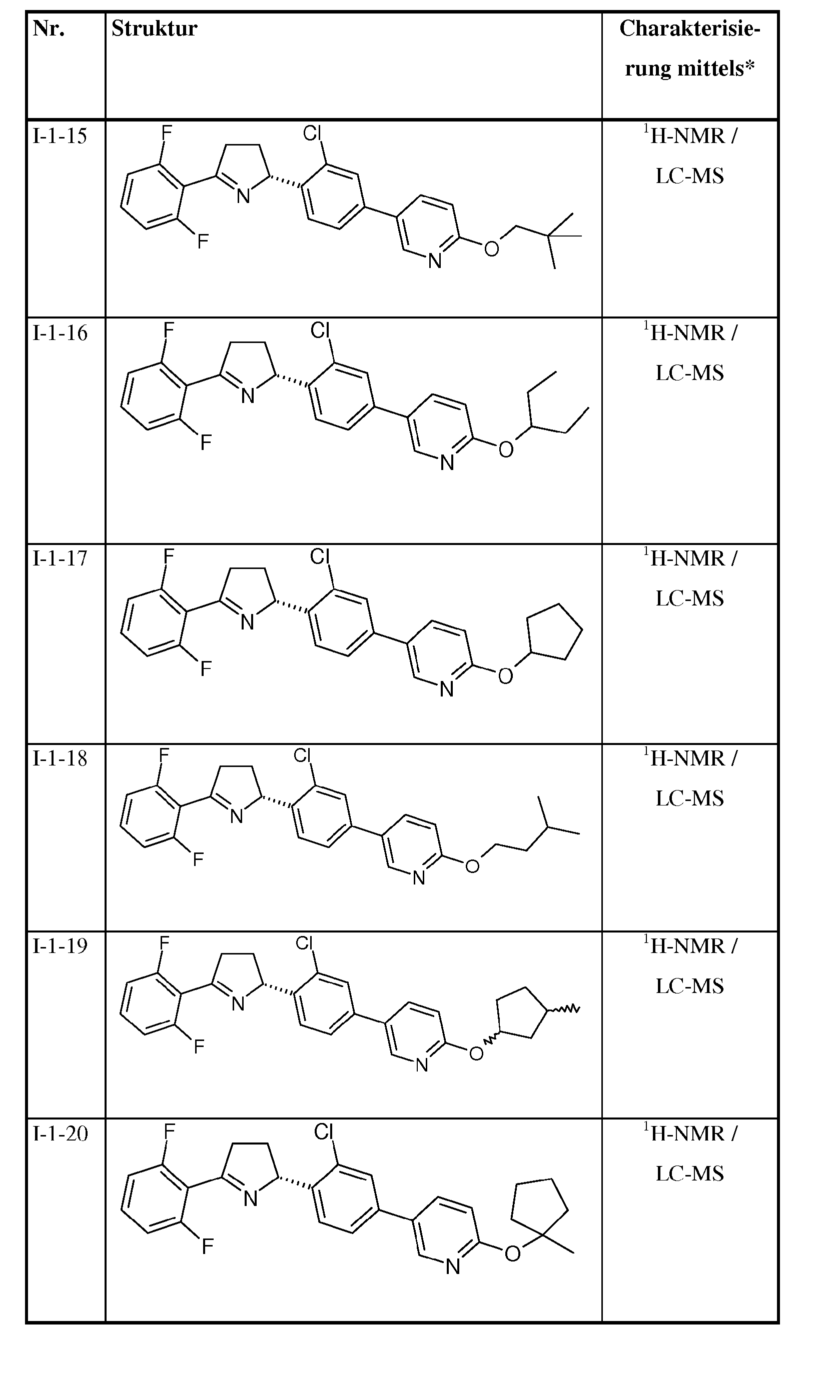

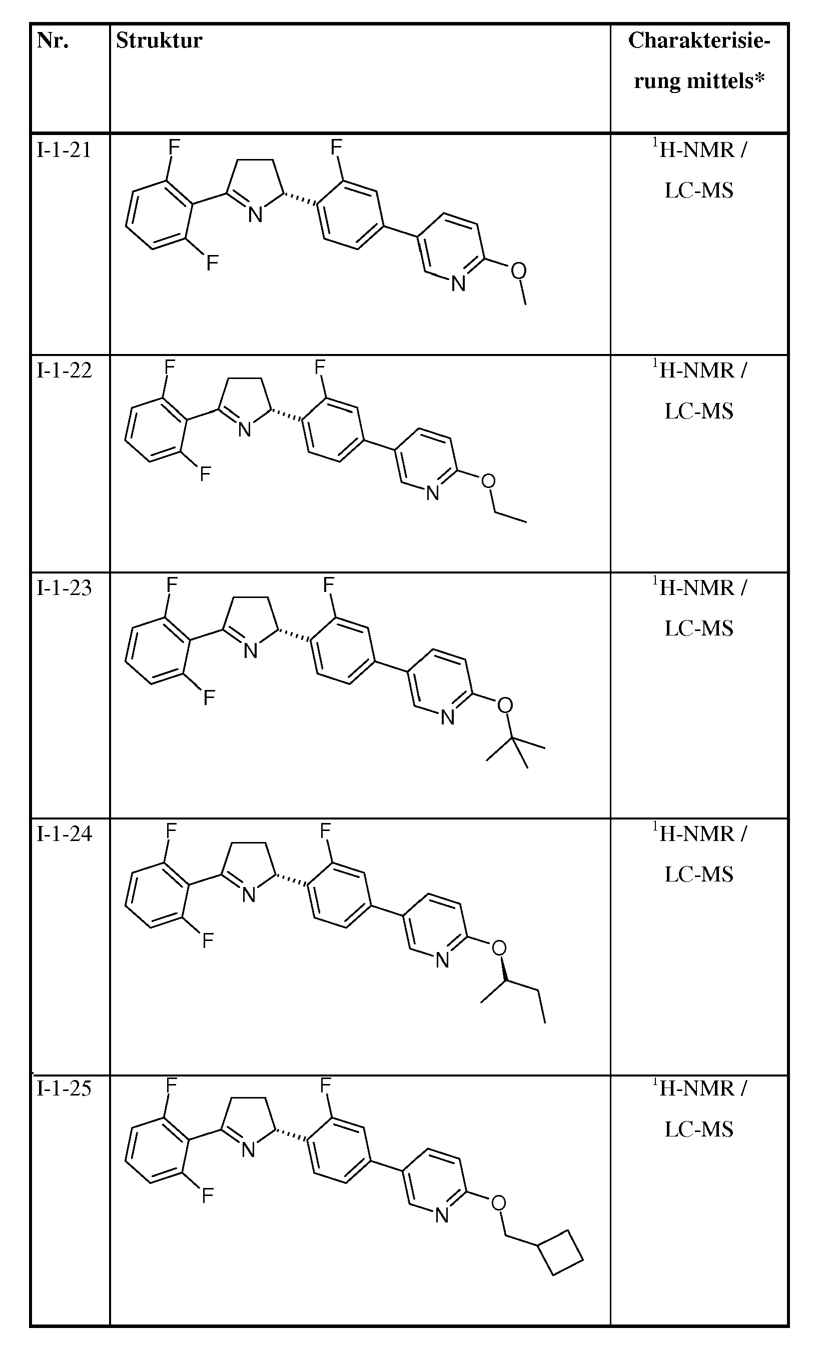

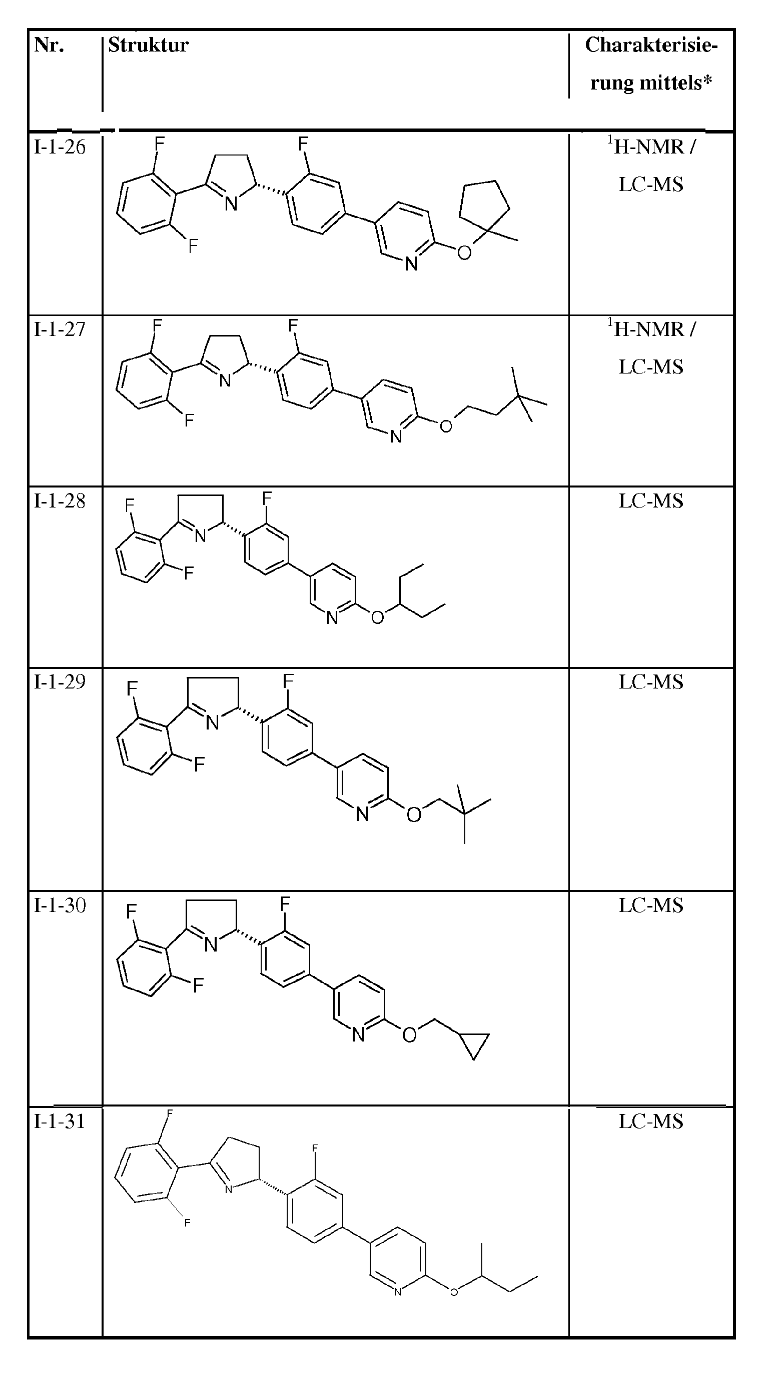

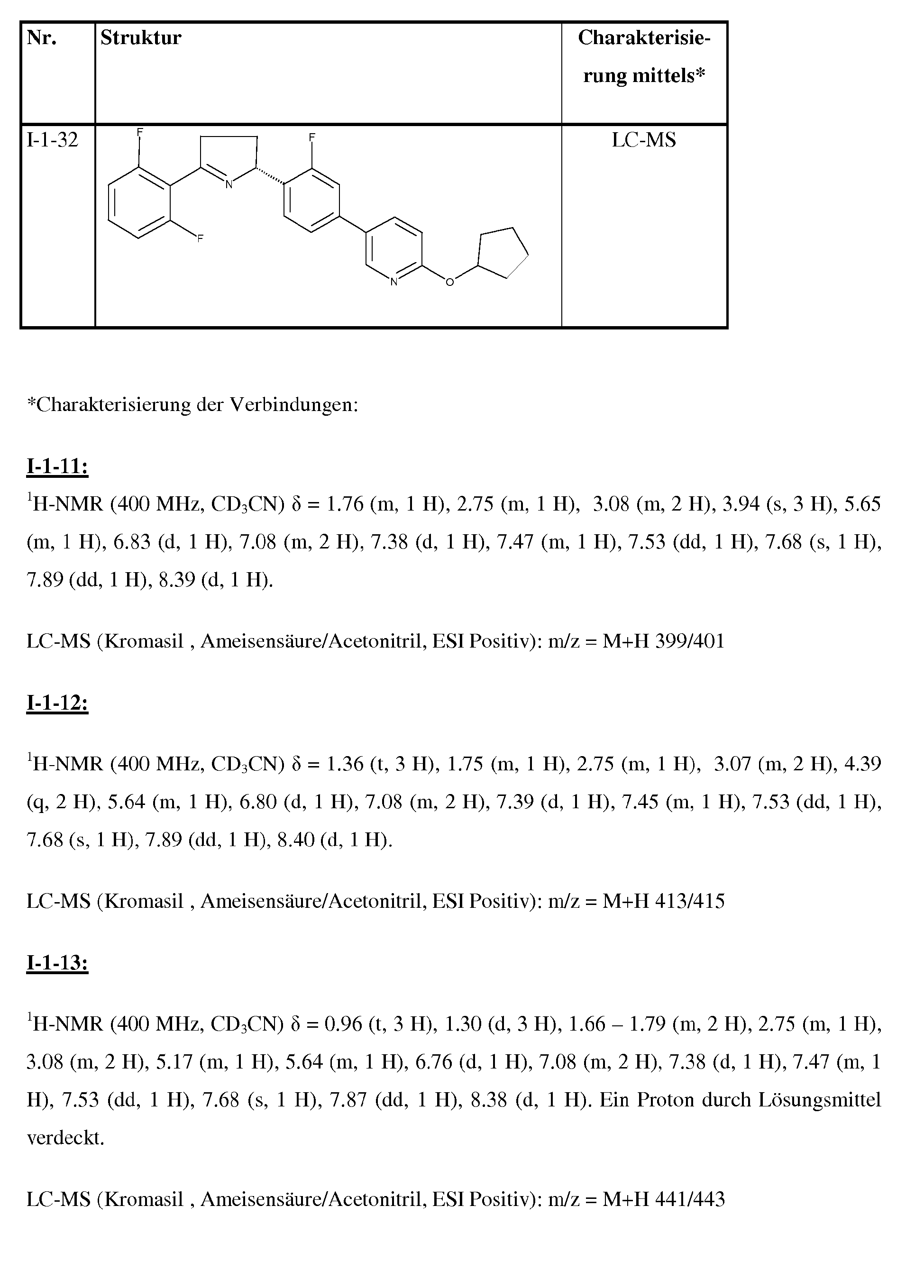

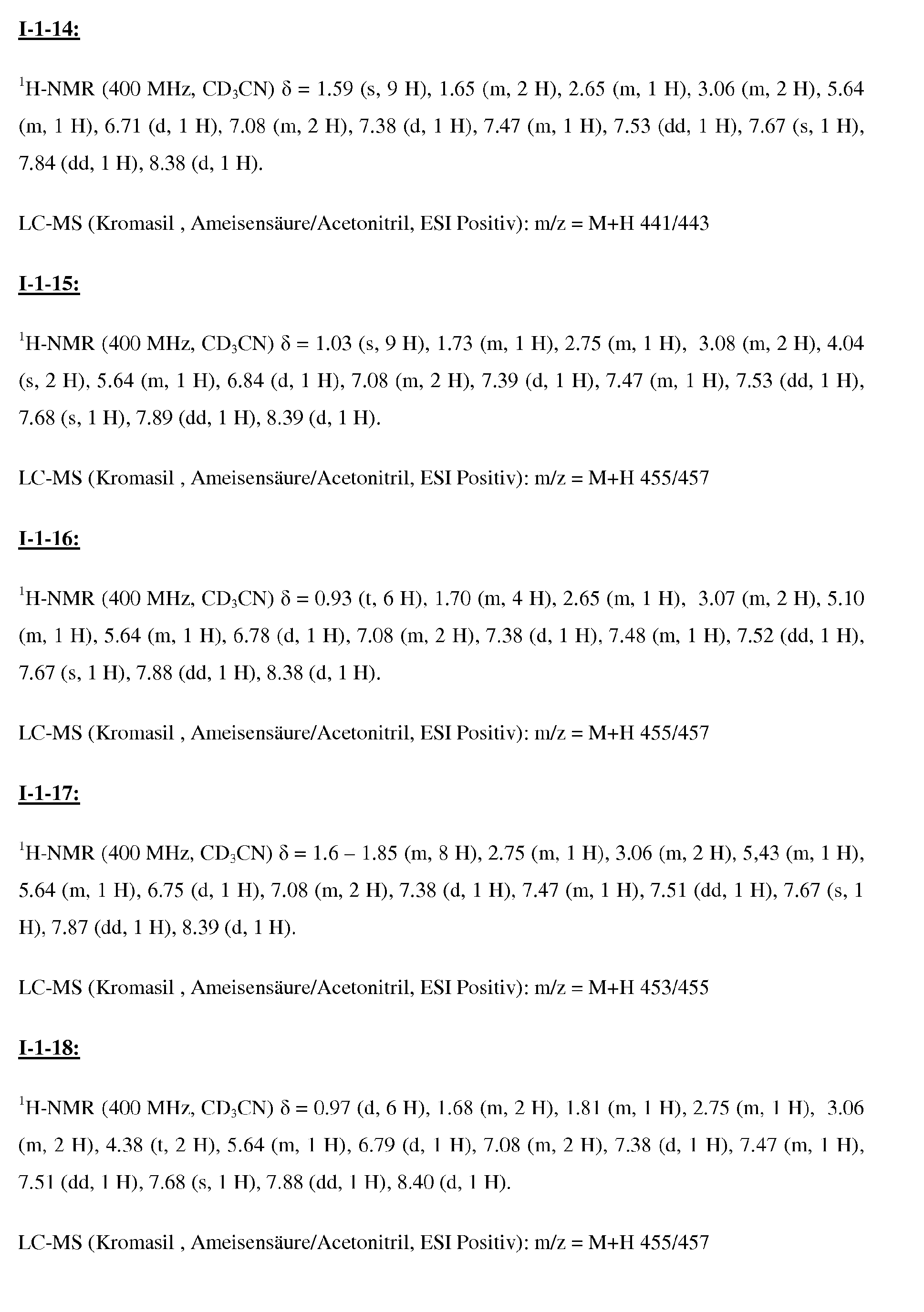

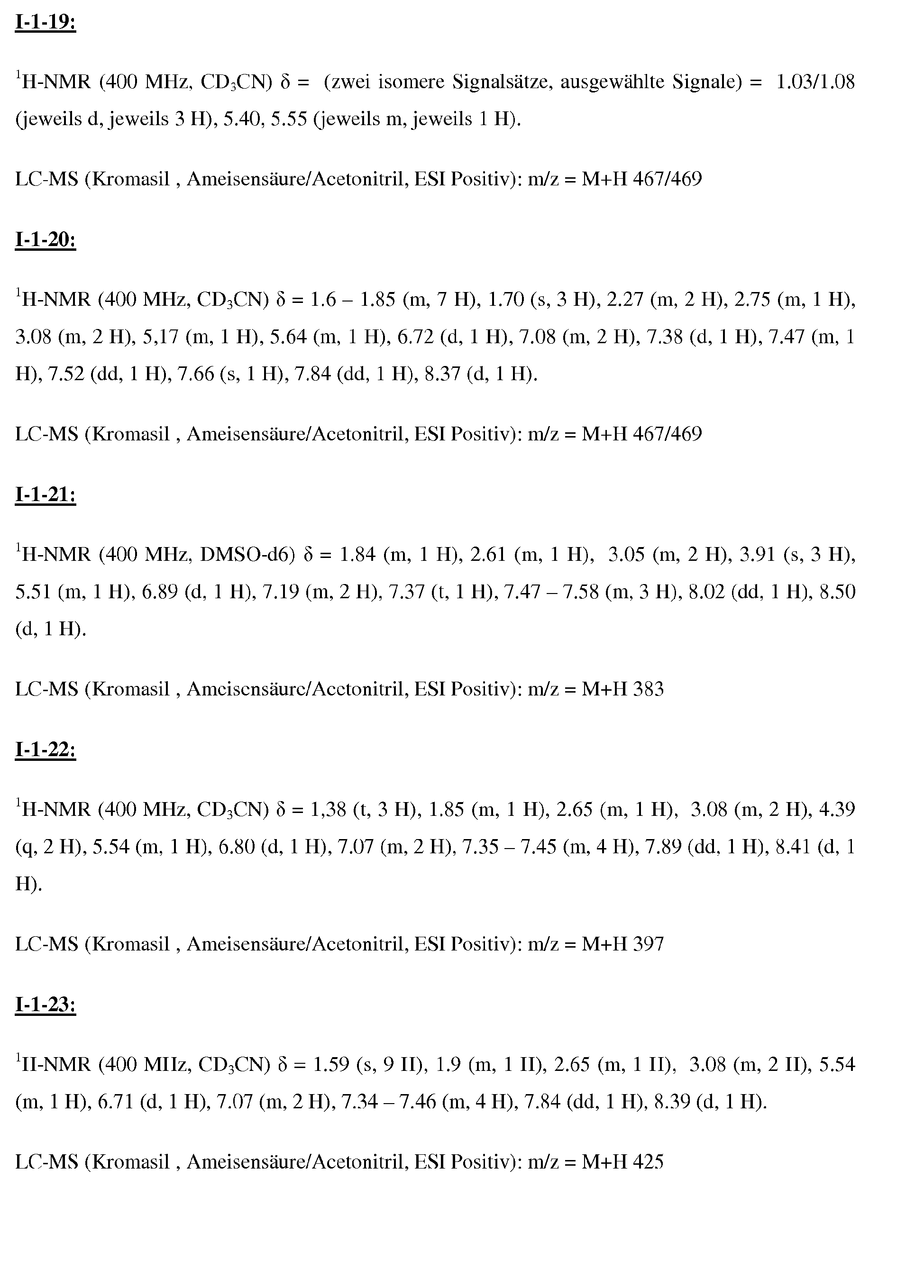

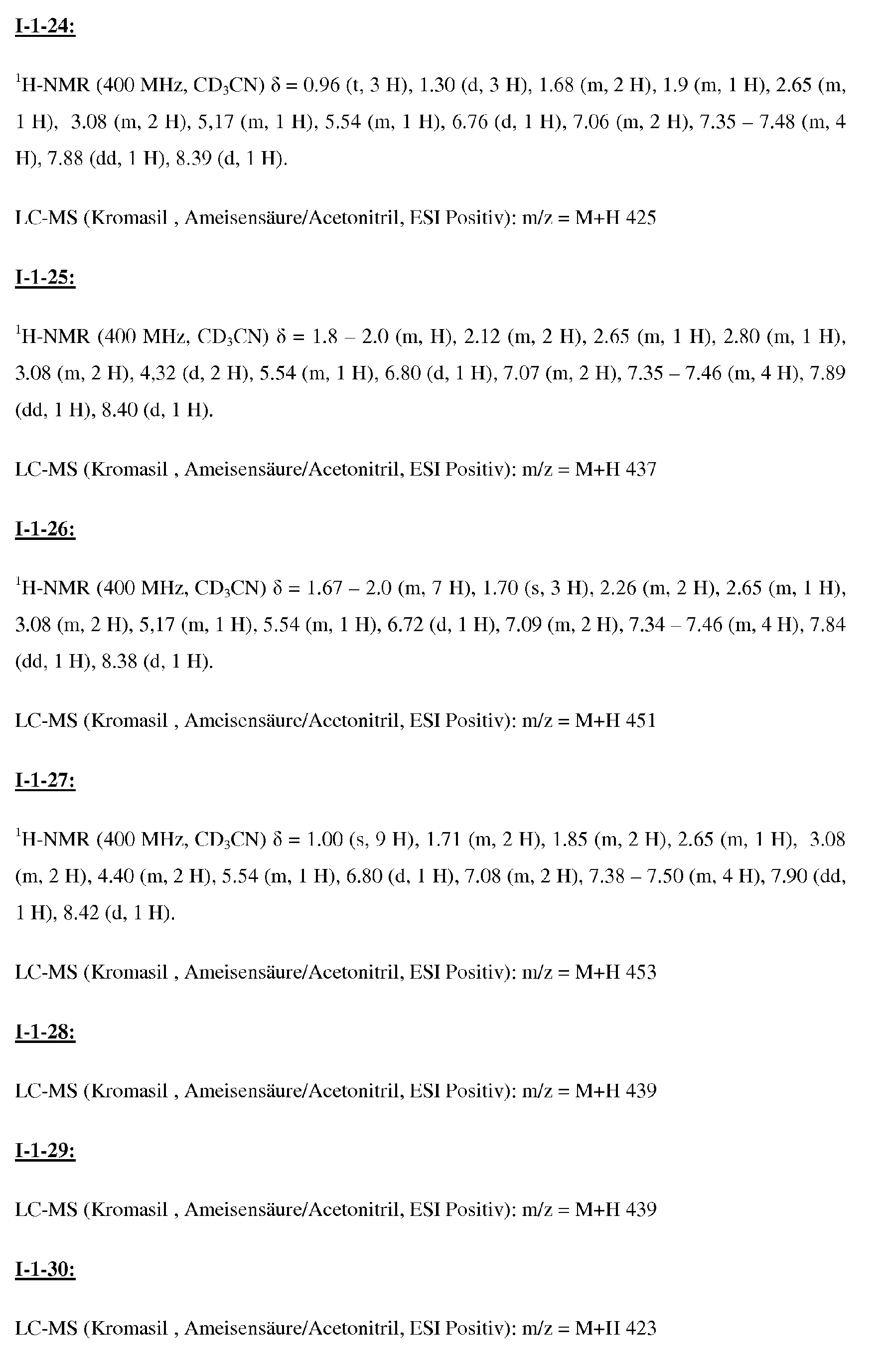

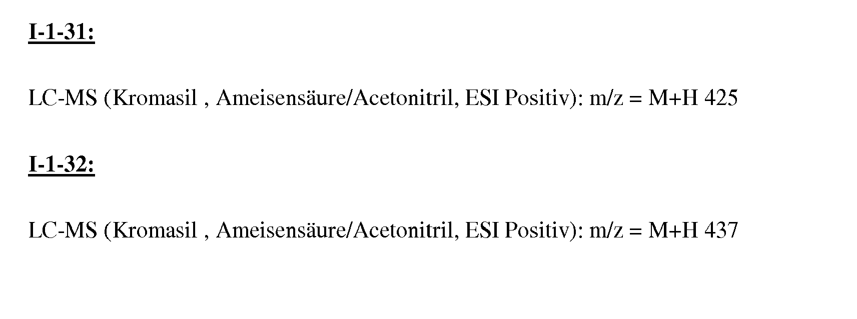

### Herstellung von Ausgangsstoffen der Formel (II-1)

### Beispiel (II-1-1)

### Stufe 1

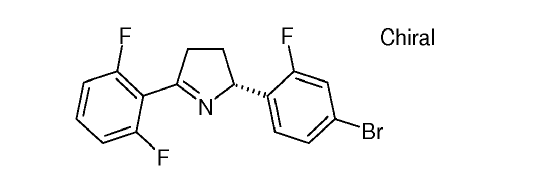

Das Racemat ist bereits bekannt (vgl. WO 04/031176). Durch Chromatographie an chiraler Phase ist das R-Enantiomer (R)-2-(4-Bromo-2-fluoro-phenyl)-5-(2,6-difluoro-phenyl)-3,4-dihydro-2*H-*pyrrole zugänglich (Bedingungen: Daicel Chiralcel OJ-RH 5 Mikrometer Säule in der Dimension 150 x 20 mm; Eluens: Wasser/Acetonitril = 20/80).

Spez. Drehwert des R-konfigurierten Enantiomeren: α_{D}²⁰= +34.2 (c= 94.4 mg/10ml Methanol).

### Stufe 2

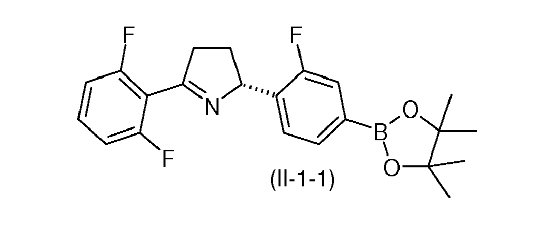

Eine Mischung aus (R)-2-(4-Bromo-2-fluoro-phenyl)-5-(2,6-difluoro-phenyl)-3,4-dihydro-2*H-*pyrrole, 23,8 g Kaliumacetat, 5.9 g1,1'-Bis(diphenylphosphino)ferrocenpalladium(II)chlorid, 4.5 g1,1'-Bis(diphenylphosphino)ferrocen und 24.8 g 4,4,5,5,4',4',5',5'-Octamethyl-2,2'-bi[1,3,2-dioxaborolanyl] in 400 ml 1,4-Dioxan wird unter Argon für 12 Stunden bei100°C gerührt.

Nach Abkühlen auf Raumtemperatur wird mit 1000 ml Wasser verdünnt und erschöpfend mit Dichlormethan extrahiert. Die vereinigten organischen Phasen ergeben nach Eindampfen und nachfolgender Chromatographie an Kieselgel mit einem Gemisch aus Cyclohexan/Essigester = 4/1 18.7 g (R)-2-[2-Fluoro-4-(4,4,5,5-tetramethyl-1,3,2-dioxaborolan-2-yl)-phenyl]-5-(2,6-difluorophenyl)-3,4-dihydro-2*H*-pyrrol.

Charakterisierung (II-1-1): Spez. Drehwert: α_{D}²⁰= +33.5 (c= 107.5 mg/10ml Methanol) ¹H-NMR (400 MHz, CD₃CN) δ = 1.32 (s, 12 H), 1.78 (m, 1 H), 2.64 (m, 1 H), 3.06 (m, 2 H), 5.53 (m, 1 H), 7.08 (m, 2 H), 7.33 - 7.50 (m, 4 H).

### Beispiel (II-1-2)

Die Verfahrensdurchführung zu Beispiel (II-1-2) erfolgt analog zu Beispiel (11-1-1).

### Stufe 1

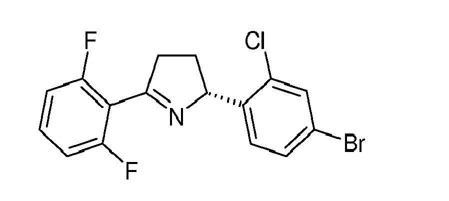

Charakterisierung von (R)-2-(4-Bromo-2-chloro-phenyl)-5-(2,6-difluoro-phenyl)-3,4-dihydro-2*H-*pyrrol: Spez. Drehwert: α_{D}²⁰= +90.7 (c= 46 mg/5 ml Methanol) ¹H-NMR (400 MHz, CD₃CN) δ = 1.69 (m, 1 H), 2.73 (m, 1 H), 3.05 (m, 2 H), 5.55 (m, 1 H), 7.07 (m, 2 H), 7.24 (d, 1 H), 7.46 (m, 1 H), 7.64 (d, 1 H).

LC-MS (Kromasil , Ameisensäure/Acetonitril, ESI Positiv): m/z = M+H 369/371/373

### Stufe 2

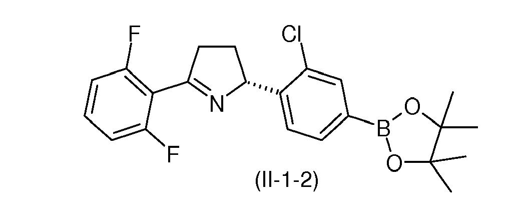

Charakterisierung von (R)-2-[2-Chloro-4-(4,4,5,5-tetramethyl-1,3,2-dioxaborolan-2-yl)-phenyl]-5-(2,6-difluoro-phenyl)-3,4-dihydro-2*H*-pyrrol (II-1-2): ¹H-NMR (400 MHz, CD₃CN) δ = 1.33 (s, 12 H), 1.72 (m, 1 H), 2.75 (m, 1 H), 3.05 (m, 2 H), 5.62 (m, 1 H), 7.07 (m, 2 H), 7.33 (d, 1 H), 7.45 (m, 1 H), 7.61 (d, 1 H), 7.71 (s, 1 H).

LC-MS (Kromasil , Ameisensäure/Acetonitril, ESI Positiv): m/z = M+H 418/420

### Herstellung von Ausgangsstoffen der Formel (III)

### Beispiel 111-1-4

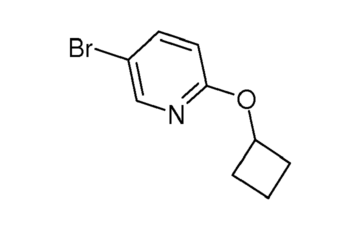

Unter Argon gibt man zu 9.1 g Cyclobutanol unter Rühren 1.9 g Natriumhydrid (60% in Öl). Nach 30 Minuten bei 50 °C werden 7.5 g 2,5-Dibrompyridin hinzugegeben und weitere 2 Stunden zum Rückfluss erhitzt (hier 130°C).

Nach Abkühlen auf Raumtemp. wird mit 100 ml Wasser versetzt und mit Dichloromethan erschöpfend extrahiert. Die getrocknete und eingedampfte organische Phase wird bei einem Druck von 1 mbar in einer Kugelrohr-Apparatur franktioniert destilliert. Die 90-120°C Fraktion ergibt nach Kristallisation aus n-Pentan 5-Bromo-2-cyclobutoxy-pyridine.

Charakterisierung von (III-1-4):

¹H-NMR (400 MHz, DMSO-d6) δ = 1.65 (m, 1 H), 1.79 (m, 1 H), 2.05 (m, 2 H), 2.38 (m, 2 H), 5.09 (quintett, 1 H), 6.76 (d, 1H), 7.84 (dd, 1 H), 8.21 (d, 1 H).

LC-MS (Kromasil , Ameisensäure/Acetonitril, ESI Positiv): m/z = M+H 228/230

Analog zu Beispiel (III-1-4) werden die folgenden Beispiele erhalten:

### Beispiel (III-1-1)

5-Bromo-2-isopropoxy-pyridine 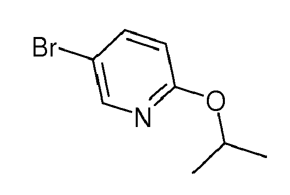 Charakterisierung von (III-1-1):

¹H-NMR (400 MHz, CDCl₃) δ = 1.33 (d, 6 H), 5.23 (heptett, 1 H), 6.59 (d, 1 H), 7.60 (dd, 1 H), 8.17 (d, 1 H).

LC-MS (Kromasil , Ameisensäure/Acetonitril, ESI Positiv): m/z = M+H 216/218

### Beispiel (III-1-2)

5-Bromo-2-propoxy-pyridine 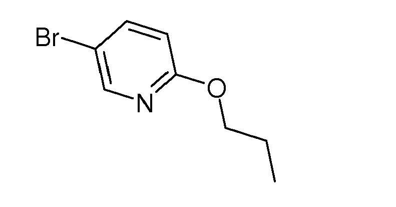 Charakterisierung von (III-1-2): ¹H-NMR (400 MHz, DMSO-d6) δ = 0.95 (t, 3 H), 1.72 (m, 2 H), 4.20 (t, 2 H), 6.78 (d, 1 H), 7.83 (dd, 1 H), 8.23 (d, 1 H).

LC-MS (Kromasil , Ameisensäure/Acetonitril, ESI Positiv): m/z = M+H 216/218

### Beispiel (III-1-3)

5-Bromo-2-butoxy-pyridine 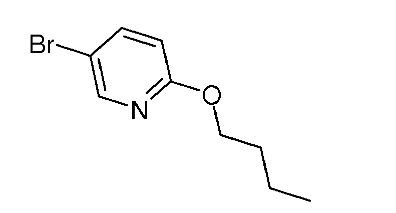 Charakterisierung von (III-1-3): ¹H-NMR (400 MHz, CD₃CN) δ = 0.95 (t, 3 H), 1.45 (m, 2 H), 1.73 (m, 2 H), 4.26 (t, 2 H), 6.68 (d, 1 H), 7.71 (dd, 1 H), 8.18 (d, 1 H).

LC-MS (Kromasil , Ameisensäure/Acetonitril, ESI Positiv): m/z = M+H 230/232

### Beispiel (111-1-5)

5-Bromo-2-pentyloxy-pyridine 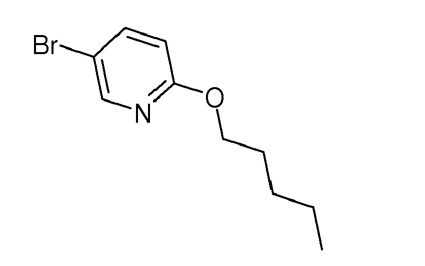 Charakterisierung von (III-1-5): ¹H-NMR (400 MHz, CD₃CN) δ = 0.91 (t, 3 H), 1.38 (m, 4 H), 1.74 (m, 2 H), 4.26 (t, 2 H), 6.67 (d, 1 H), 7.71 (dd, 1 H), 8.18 (d, 1 H).

LC-MS (Kromasil , Ameisensäure/Acetonitril, ESI Positiv): m/z = M+H 244/246

Analog dazu werden die folgenden Beispiele III-2-1 bis III-2-5 erhalten.

### Beispiel (III-2-1)

5-Chloro-2-isopropoxy-pyridine 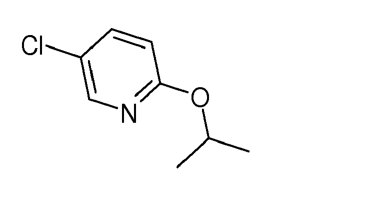 Charakterisierung von (III-2-1): ¹H-NMR (400 MHz, DMSO-d6) δ = 1.28 (d, 6 H), 5.19 (heptett, 1 H), 6,76 (d, 1 H), 7.72 (dd, 1 H), 8.15 (d, 1 H).

### Beispiel (III-2-2)

5-Chloro-2-propoxy-pyridine 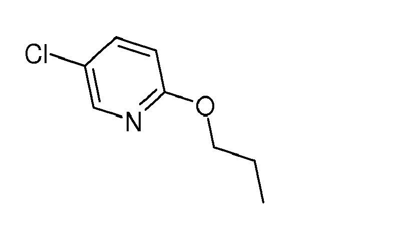 Charakterisierung von (III-2-2): ¹H-NMR (400 MHz, DMSO-d6) δ = 0.92 (t, 3 H), 1.71 (m, 2 H), 4.19 (t, 2 H), 6.82 (d, 1 H), 7.74 (dd, 1 H), 8.15 (d, 1 H).

### Beispiel (III-2-3)

5-Chloro-2-butoxy-pyridine 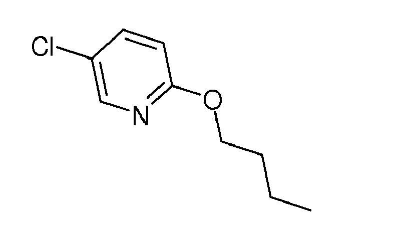 Charakterisierung von (III-2-3): ¹H-NMR (400 MHz, DMSO-d6) δ = 0.89 (t, 3 H), 1.41 (m, 2 H), 1.69 (m, 2 H), 4.25 (t, 2 H), 6.62 (d, 1 H), 7.74 (dd, 1 H), 8.15 (d, 1 H).

### Beispiel (III-2-4)

5-Chloro-2-cyclobutoxy-pyridine 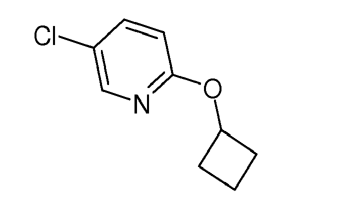 Charakterisierung von (III-2-4): ¹H-NMR (400 MHz, DMSO-d6) δ = 1.54 (m, 1 H), 1.76 (m, 1 H), 2.05 (m, 2 H), 2.39 (m, 2 H), 5.10 (m, 1 H), 6.79 (d, 1H), 7.74 (dd, 1 H), 8.14 (d, 1 H).

### Beispiel (III-2-5)

5-Chloro-2-pentyloxy-pyridine 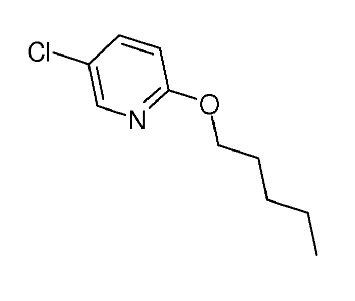 Charakterisierung von (III-2-5): ¹H-NMR (400 MHz, DMSO-d6) δ = 0.89 (t, 3 H), 1.35 (m, 4 H), 1.70 (m, 2 H), 4.24 (t, 2 H), 6.82 (d, 1 H), 7.74 (dd, 1 H), 8.15 (d, 1 H).

Die Bestimmung der voranstehend angegebenen ¹H-NMR-Daten erfolgt mit einem Bruker Avance 400, mit Tetramethylsilan als Referenz (0.0 ppm) und den Lösungsmitteln CD₃CN, d6-DMSO oder CDCl₃. Die Charakterisierung der Signalaufspaltung erfolgt mit s = Singulett, d = Duplett, t=triplett, q=Quartett, und m=Multiplett.

Die Bestimmung mit der LC-MS im sauren Bereich erfolgt bei pH 2,7 mit 0,1%wässriger Ameisensäure und Acetonitril als Eluenten.

### Anwendungsbeispiele

### Beispiel A

### Spodoptera frugiperda -Test (SPODFR Sprühapplication)

| | |
|---|---|
| Lösungsdmittel: | 78.0 Gewichtsteile Aceton |
| | 1.5 Gewichtsteile Dimethylformamid |
| Emulgator: | 0.5 Gewichtsteile Alkylarylpolyglcolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Mais(*Zea mais*)-Blattteile werden mit einer Wirkstoffzubereitung der gewünschten Konzentration besprüht. Sobald es abgetrocknet ist, werden die Blattteile mit Raupen des Heerwurms (Spodoptera frugiperda) besetzt.

Nach der gewünschten Zeit wird die Abtötung in % bestimmt. Dabei bedeutet 100%, dass alle Raupen abgetötet wurden; 0% bedeutet, dass keine Raupen abgetötet wurden.

Bei diesem Test zeigen z. B. die folgenden in der Tabelle A aufgeführten erfindungsgemäßen Wirkstoffe überlegene Wirksamkeit gegenüber dem Stand der Technik.

**Tabelle A:**

| Wirkstoffe | Konzentration in g/ha | Abtötungsgrad in % / 7 d |
|---|---|---|
| (I-1-5) | 0,032 | 83 |
| 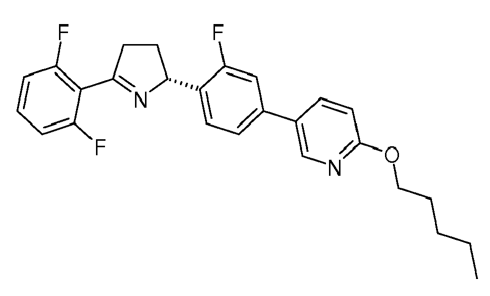 | | |
| erfindungsgemäß | | |
| 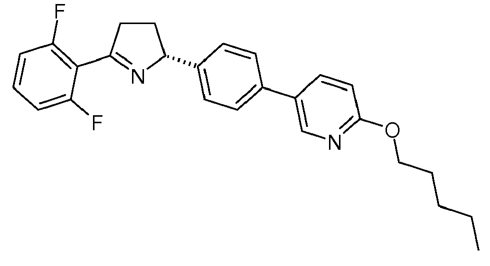 | 0,032 | 0 |
| bekannt | | |
| (I-1-4) | 0,8 | 83 |
| 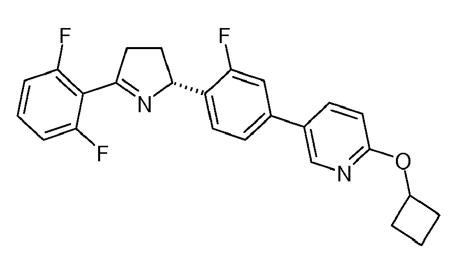 | | |
| erfindungsgemäß | | |
| 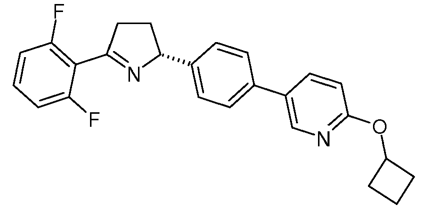 | 0,8 | 33 |
| bekannt | | |
| (I-1-3) | 0,032 | 67 |
| 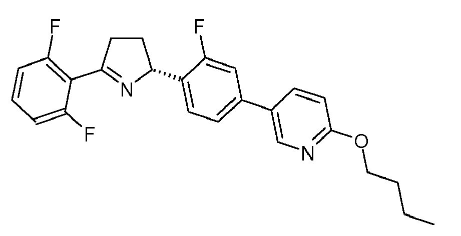 | | |
| erfindungsgemäß | | |
| 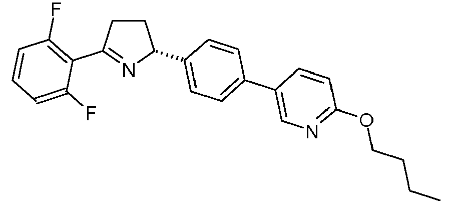 | 0,032 | 17 |
| bekannt | | |
| (I-1-2) | 0,8 | 100 |
| 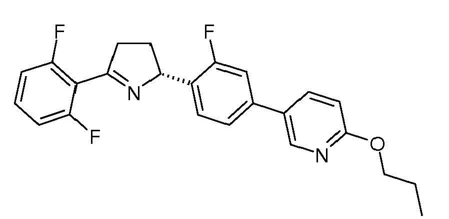 | | |
| erfindungsgemäß | | |
| 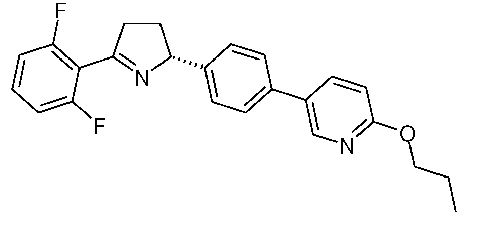 | 0,8 | 33 |
| bekannt | | |
| (I-1-7) | 0,8 | 100 |
| | | |
| 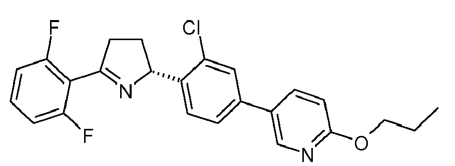 | 0,16 | 100 |
| erfindungsgemäß | | |
| (I-1-6) | 0,8 | 100 |
| | 0,16 | 100 |
| 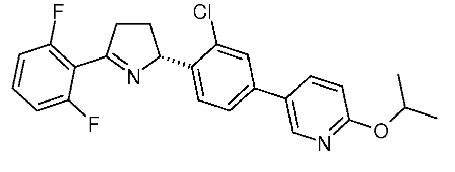 | | |
| erfindungsgemäß | | |
| 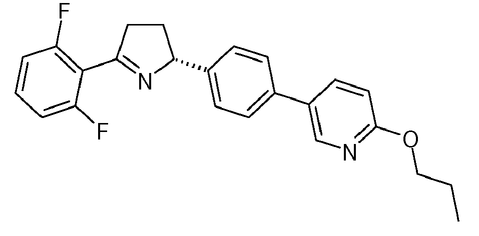 | 0,8 | 33 |
| | 0,16 | 33 |
| bekannt | | |

### Beispiel B

**Spodoptera exigua-Test** (SPODEX)

| | |
|---|---|
| Lösungsmittel: | 7 Gewichtsteile Dimethylformamid |
| Emulgator: | 2 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen der Zuckerrübeneule (Spodoptera exigua) besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

Bei diesem Test zeigen z. B. die folgenden in der Tabelle B aufgeführten erfindungsgemäßen Wirkstoffe überlegene Wirksamkeit gegenüber dem Stand der Technik.

**Tabelle B:**

| Wirkstoffe | Konzentration in ppm | Abtötungsgrad in % / 7 d |
|---|---|---|
| (1-1-1) | 4 | 100 |
| 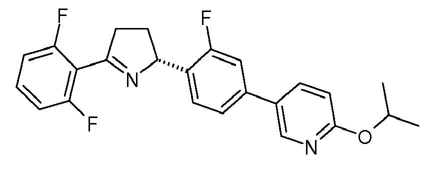 | | |
| erfindungsgemäß | | |
| 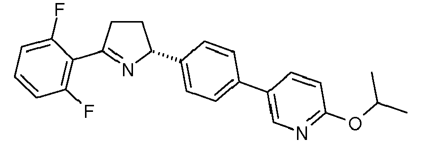 | 4 | 20 |
| bekannt | | |

### Beispiel C

### Lucilia cuprina-Test (LUCICU)

Lösungsmittel: Dimethylsulfoxid

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße, die Pferdefleisch enthalten, das mit der Wirkstoffzubereitung der gewünschten Konzentration behandelt wurde, werden mit *Lucilia cuprina* Larven besetzt.

Nach der gewünschten Zeit wird die Abtötung in % bestimmt. Dabei bedeutet 100 %, dass alle Larven abgetötet wurden; 0 % bedeutet, dass keine Larven abgetötet wurden.

Bei diesem Test zeigen z. B. die folgenden Verbindungen der Herstellungsbeispiele eine Wirkung von ≥ 80 % bei einer Aufwandmenge von 100 ppm:
Bsp. Nr. I-1-1, I-1-2, I-1-3, I-1-5, I-1-6, I-1-7

### Beispiel D

### Boophilus microplus -Test (BOOPMI Injektion)

Lösungsmittel: Dimethylsulfoxid

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Die Wirkstofflösung wird in das Abdomen *(Boophilus microplus)* injiziert, die Tiere werden in Schalen überführt und in einem klimatisierten Raum aufbewahrt.

Nach der gewünschten Zeit wird die Wirkung in % bestimmt. Dabei bedeutet 100%, dass keine Zecke fertile Eier gelegt hat.

Bei diesem Test zeigen z. B. die folgenden Verbindungen der Herstellungsbeispiele eine Wirkung von ≥ 80 % bei einer Aufwandmenge von 20 µg/Tier:
Bsp. Nr. I-1-2, I-1-3, I-1-5, I-1-6, I-1-7

### Beispiel E

### Amblyomma hebaraeum -Test (AMBYHE)

Lösungsmittel: Dimethylsulfoxid

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Zeckenymphen *(Amblyomma hebraeum*) werden in perforierte Plastikbecher gesetzt und in der gewünschten Konzentration eine Minute getaucht. Die Zecken werden auf Filterpapier in eine Petrischale überführt und in einem Klimaschrank 42 Tage gelagert.

Nach der gewünschten Zeit wird die Abtötung in % bestimmt. Dabei bedeutet 100 %, dass alle Zecken abgetötet wurden; 0 % bedeutet, dass keine Zecken abgetötet wurden.

Bei diesem Test zeigen z. B. die folgenden Verbindungen der Herstellungsbeispiele eine Wirkung von ≥ 80 % bei einer Aufwandmenge von 100 ppm:
Bsp. Nr. I-1-2, I-1-5, I-1-7

### Beispiel F

### Boophilus microplus -Test (BOOPMI dip)

Lösungsmittel: Dimethylsulfoxid

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Adulte Zeckenweibchen *(Boophilus microplus)* werden in perforierte Plastikbecher gesetzt und in der gewünschten Konzentration eine Minute getaucht. Die Zecken werden auf Filterpapier in Schalen überführt. Die Zecken werden 42 Tage unter klimatisierten Bedingungen gelagert und die Eiablage beobachtet.

Nach der gewünschten Zeit wird die Wirkung in % bestimmt. Dabei bedeutet 100%, dass keine Zecke fertile Eier gelegt hat.

Bei diesem Test zeigen z. B. die folgenden Verbindungen der Herstellungsbeispiele eine Wirkung von ≥ 80 % bei einer Aufwandmenge von 100 ppm:
Bsp. Nr. I-1-5, I-1-7

## Patentansprüche

1. Δ¹-Pyrroline der Formel (I) 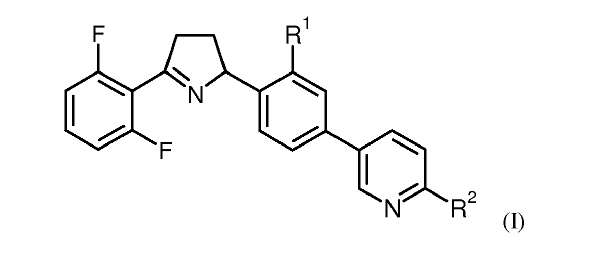 in welcher
R¹ für Halogen steht,
R² für jeweils gegebenenfalls substituiertes Alkyl, Alkenyl, Alkoxy, Alkenyloxy; oder für jeweils gegebenenfalls substituiertes Cycloalkyl, Cycloalkoxy steht.

2. Δ¹-Pyrroline der Formel (I-1) 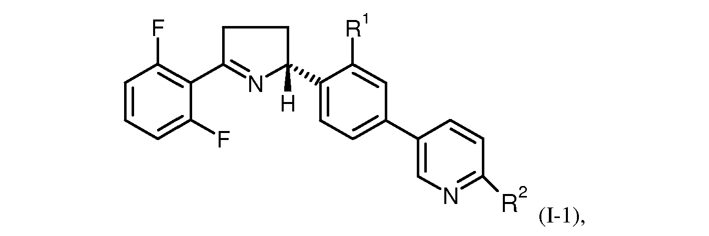 in welcher R¹ und R² die in Anspruch 1 angegebenen Bedeutungen haben.

3. Verfahren zur Herstellung von Verbindungen der Formel (I) gemäß Anspruch 1, **dadurch gekennzeichnet, dass** man Δ¹-Pyrroline der Formel (II) 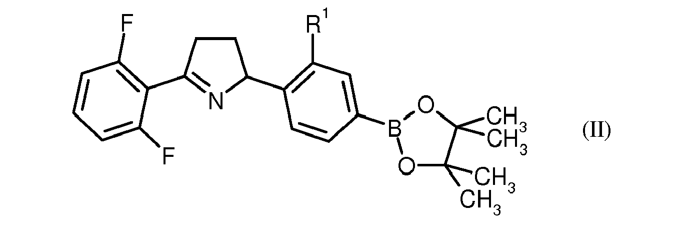 in welcher R¹ die in Anspruch 1 angegebenen Bedeutungen hat, mit Pyridinen der Formel (III) 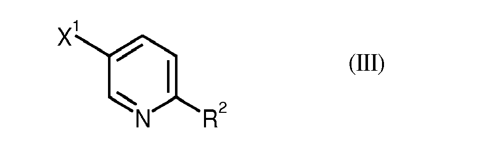 in welcher
R² die in Anspruch 1 angegebenen Bedeutungen hat und
X¹ für Brom, Chlor, oder Jod steht,
in Gegenwart eines Katalysators und gegebenenfalls in Gegenwart eines Verdünnungsmittels umsetzt.

4. Schädlingsbekämpfungsmittel, **gekennzeichnet durch** einen Gehalt an mindestens einer Verbindung der Formel (I) gemäß Anspruch 1 neben Streckmitteln und/oder oberflächenaktiven Stoffen.

5. Verwendung von Verbindungen der Formel (I) gemäß Anspruch 1 zur Bekämpfung von Schädlingen im Pflanzenschutz, im Materialschutz und/oder im veterinärmedizinischen Sektor.

6. Verfahren zur Bekämpfung von Schädlingen, **dadurch gekennzeichnet, dass** man Verbindungen der Formel (I) gemäß Anspruch 1 auf Schädlinge und/oder ihren Lebensraum einwirken lässt.

7. Verfahren zur Herstellung von Schädlingsbekämpfungsmitteln, **dadurch gekennzeichnet, dass** man Verbindungen der Formel (I) gemäß Anspruch 1 mit Streckmitteln und/oder oberflächenaktiven Stoffen vermischt.
